# EUROPÄISCHE PATENTSCHRIFT

(11) **EP 3 204 111 B1**
(45) Veröffentlichungstag und Bekanntmachung des Hinweises auf die Patenterteilung: **10.07.2019**
(21) Anmeldenummer: 15777680.8
(22) Anmeldetag: 07.10.2015
(51) Int. Cl.: A61N 1/36, A61N 1/05

(54) **IMPLANTIERBARE ANORDNUNG**
IMPLANTABLE ASSEMBLY
ENSEMBLE IMPLANTABLE

(30) Priorität: 07.10.2014 DE 102014014942
(43) Veröffentlichungstag der Anmeldung: 16.08.2017
(73) Patentinhaber: Neuroloop GmbH, 79108 Freiburg (DE)
(72) Erfinder: PLACHTA, Dennis, 79279 Vörstetten (DE); GIERTHMÜHLEN, Mortimer, 79104 Freiburg (DE); STIEGLITZ, Thomas, 79110 Freiburg (DE); ZENTNER, Josef, 79117 Freiburg (DE)
(74) Vertreter: Rösler, Uwe
(86) Internationale Anmeldenummer: PCT/EP2015/073131
(87) Internationale Veröffentlichungsnummer: WO 2016/055513

(56) Entgegenhaltungen:
- US-A1- 2006 122 675
- US-A1- 2010 204 741
- DENNIS T T PLACHTA / OSCAR COTA / THOMAS STIEGLITZ / MORTIMER GIERTHMUEHLEN: "Selektive Ableitung und Stimulation fuer ein blutdrucksenkendes Implantat unter Verwendung von Vielkanal-Cuff-Elektroden", TECHNISCHES MESSEN TM, R.OLDENBOURG VERLAG. MUNCHEN, DE, Bd. 80, Nr. 5, 1. Januar 2013 (2013-01-01) , Seiten 163-172, XP009187692, ISSN: 0171-8096

## Beschreibung

### Technisches Gebiet

Die Erfindung bezieht sich auf eine implantierbare Anordnung zur ortsselektiven Erfassung neuronaler elektrischer Signale, die sich längs wenigstens einer in einem Nervenfaserbündel enthaltenden Nervenfaser ausbreiten, sowie zur selektiven elektrischen Stimulation der wenigstens einen Nervenfaser. Die implantierbare Anordnung umfasst eine um ein Nervenfaserbündel manschettenartig anlegbare, implantierbare Elektrodenanordnung, mit der innerhalb des Nervenfaserbündels selektierte Nervenfasern mit elektrischen Signalen beaufschlagbar sind. Die elektrische Stimulation erfolgt insbesondere zur gezielten Beeinflussung des Blutdruckes in einem tierischen oder menschlichen Patienten.

### Stand der Technik

Die arterielle Hypertonie ist eine weltweit verbreitete, typische Zivilisationskrankheit, die das Leben von Millionen Patienten bedroht und gleichzeitig im hohen Maße die Gesundheitssysteme belastet. Bisher bekannte therapeutische Maßnahmen gründen auf der Verabreichung blutdrucksenkender Arzneimittel, wie bspw. ACE-Hemmer, Betablocker etc., diese weisen jedoch neben der erwünschten blutdrucksenkenden Wirkung beachtliche Nebenwirkungen auf, wie bspw. Bradykardie, Herzinsuffizienz, Asthmaanfälle etc. auf. Hinzukommt, dass trotz der Entwicklung neuer blutdrucksenkender Medikamente bei bis zu 30% aller Patienten bei entsprechender Medikation kein adäquater Zielblutdruck erreicht werden kann, siehe Beitrag von H. R. Black, et al., "Principal results of the controlled onset Verapamil investigation of cardiovascular end points (Convince), TRIAL. Jama, 289 (16), S. 2073 - 2082), 2003.

Einen anderen therapeutischen Ansatz zur Begegnung von Bluthochdruck verfolgt eine Studie seitens der Anmelderin, die in dem Artikel von Dennis T. T. Plachta, Oscar Cota, Thomas Stieglitz, Mortimer Gierthmuehlen, "Selektive Ableitung und Stimulation für ein blutdrucksenkendes Implantat unter Verwendung von Vielkanal-Cuff-Elektroden", tm - Technisches Messen, 2013, Vol.80 (5), pp.163-172 publiziert worden ist. Die anhand von an Ratten durchgeführten Tierversuchen gewonnenen Erkenntnisse begründen die Möglichkeit mittels einer an einem Nervenfaserbündelabschnitt des Nervus Vagus implantierten Elektrodenanordnung neuronale elektrische Signale ortsaufgelöst aus dem Nervenfaserbündelabschnitt zu detektieren sowie elektrische Signale an selektierte Nervenfasern zu deren Stimulierung zu Zwecken einer technisch initiierten Blutdruckreduzierung zu applizieren. Eine derartige Vagusnervstimulation besitzt somit grundsätzlich das Potential als Alternative zur Behandlung des therapierefraktären Blutdruckes etabliert zu werden.

Das Konzept der selektiven Vagusnervstimulation stützt sich auf Erfahrungen der seit vielen Jahren angewandten und etablierten neuromodulatorischen Therapie schwerer Formen der Epilepsie, bei der der Vagusnerv mit Hilfe einer implantierten Elektrodenanordnung gesamtheitlich elektrisch stimuliert wird, um bei sich anbahnenden epileptischen Anfällen zumindest deren Ausmaß in Hinblick auf Stärke und zeitliche Dauer abzumildern, siehe hierzu F. Sidiqui, et al., "Cumulative effect of Vagus nerve stimulators on intractable seizures observed over a period of 3 years", Epilepsy and Behavior, 18(3), S. 299 - 302, 2010 sowie T. Stieglitz, "Neuroprothetik und Neuromodulation - Forschungsansätze und klinische Praxis bei Therapie und Rehabilitation", Bundesgesundheitsblatt - Gesundheitsforschung - Gesundheitsschutz, 53(8), S. 783 - 790, 2010.

Im Gegensatz dazu gilt es zur chronischen Behandlung von Hypertonie die blutdruckrelevanten Fasern messtechnisch zunächst zu lokalisieren um sie anschließend in geeigneter Weise selektiv elektrisch zu stimulieren. Um den Vagusnerv durch die implantive Maßnahme der Applikation einer Elektrodenanordnung möglichst zu schonen und das Epineurium des Vagusnervs möglichst nicht zu irritieren, wird in dem zitierten Beitrag von Dennis T. T. Plachta et al. der Einsatz einer sog. Cuff-Elektrode vorgeschlagen, die extraneural am Vagusnerv anbringbar ist. Dies hat den Vorteil einer relativ leichten Positionierung der Cuff-Elektrode längs des Vagusnerves und ermöglicht darüber hinaus einen geringfügig invasiv und daher schonend und schnell durchzuführenden chirurgischen Eingriff am Patienten.

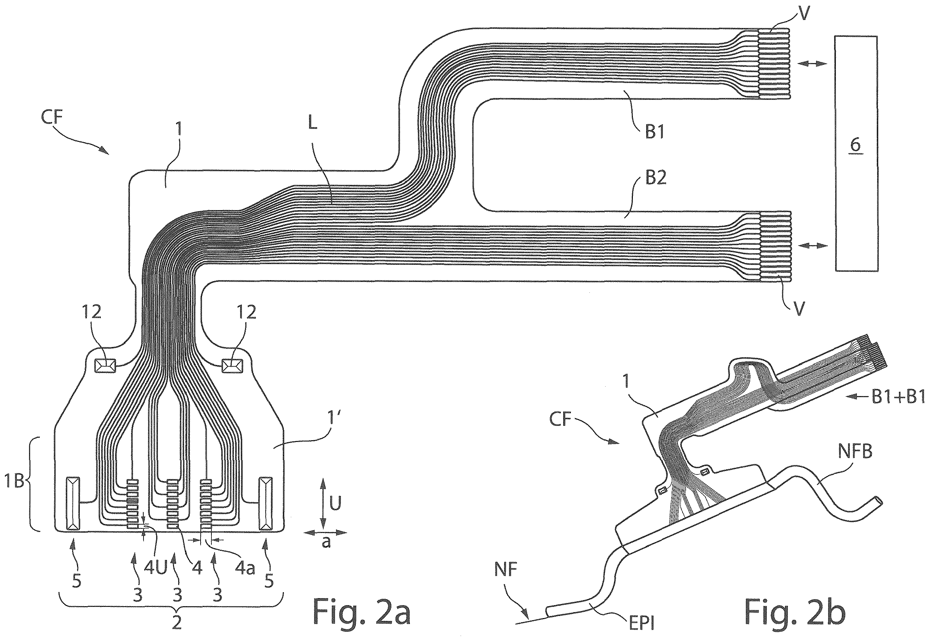

Für die natürliche Blutdruckregelung dient der Baroreflex, der einen homöostatischen, selbstregulierenden Mechanismus darstellt und bei einem erhöhten Blutdruck reflektorisch unterschiedliche Effektoren aktiviert. U.a. wird hierbei die Herzfrequenz abgesenkt, aber auch die arteriellen Gefäße werden geweitet, um damit den Blutdruck zu senken. Im Falle eines niedrigen Blutdruckes wird der Baroreflex unterdrückt, wodurch die Herzfrequenz steigt und Blutgefäße verengt werden damit der Blutdruck wieder steigt. Die sensorischen Eingänge für den Baroreflex stellen sog. Barorezeptoren dar, die sich unter anderem in den Wänden des Aortenbogens befinden. Von dort ziehen die Blutdruckinformationen monosynaptisch längs der blutdruckrelevanten Nervenfasern, im Folgenden als barorezeptive Fasern bezeichnet, in den Hirnstamm. Beim Überschreiten eines Schwellenwertes für den Blutdruck löst der Baroreflex eine Inhibition sympathischer Nervenfasern aus, was zu einer unmittelbaren Absenkung des Blutdruckes führt. Mit Hilfe der unter Bezugnahme auf Figuren 2a, b dargestellten Manschettenelektrode, die in der englischsprachigen Literatur häufig als Cuff-Elektrode bezeichnet wird, ist es möglich, diesen Baroreflexmechanismus zu nutzen, indem die den Hirnstamm zugeführten Druckinformationen selektiv detektiert und gleichfalls selektiv "überschrieben" werden, um den Hirnstamm auf diese Weise eine wesentlich erhöhte Blutdrucksituation zu suggerieren, wodurch eine natürliche signifikante Blutdruckabsenkung initiiert wird.

Figur 2a zeigt die bekannte Manschettenelektrode E in einer flächigen Draufsicht in einem planar entfalteten Zustand. Figur 2b zeigt die Manschettenelektrode E im implantierten Zustand, in dem Bereiche B1, B2 der Manschettenelektrode E zu Zwecken einer raumsparenden Form aufeinander gefaltet sind und darüber hinaus ein mit einer ersten Elektrodenanordnung 2 versehener Trägersubstratbereich 1B der Manschettenelektrode E manschettenartig einen Bereich eines Nervenfaserbündels NFB umfasst.

Die Manschettenelektrode E besteht aus einem flexiblen, biokompatiblen Trägersubstrat 1, das in der realisierten Ausführungsform eine ca. 11 µm dicke Polyimid-Folie ist, an deren der Zeichenebene in Figur 2a zugewandten Trägersubstratoberseite zu Zwecken der ortsaufgelösten Erfassung neuronaler elektrischer Signale sowie auch zur selektiven elektrischen Stimulation einzelner im Nervenfaserbündel NFB verlaufender Nervenfasern NF eine aus einer Vielzahl einzelner Elektroden zusammengesetzte erste Elektrodenanordnung 2 aufgebracht ist. Die einzelnen Elektroden der ersten Elektrodenanordnung 2 gelangen in unmittelbaren Oberflächenkontakt mit dem Epineurium EPI des Nervenfaserbündels NFB, da sich das Trägersubstrat 1 im Trägersubstratbereich 1B durch entsprechende Einprägung einer mechanischen Folienvorspannung selbständig unter Ausbildung einer dem Nervenfaserbünde NFB zugewandt orientierten geradzylinderförmigen Trägersubstratoberfläche 1' aufrollt, wie dies in Figur 2b ersichtlich ist. So nehmen die einzelnen Elektroden der ersten Elektrodenanordnung 2 eine in Umfangsrichtung U um das Nervenfaserbündel NFB gebogene ringförmige Raumform an.

Sowohl zur ortsselektiven Erfassung neuronaler elektrischer Signale als auch zur selektiven elektrischen Stimulation wenigstens einer Nervenfaser NF dienen drei axial jeweils gleich zueinander beabstandet angeordnete, erste Elektrodenstrukturen 3, die in Umfangsrichtung U wenigstens zwei, im illustrierten Ausführungsbeispiel gemäß Figur 2a, b jeweils acht erste Elektrodenflächen 4 umfassen. Die jeweils acht einer ersten Elektrodenstruktur 3 zugehörigen ersten Elektrodenflächen 4 sind in Umfangsrichtung U gleich verteilt, d. h. in 45° Winkelabständen, angeordnet. Dies ermöglicht eine achtfache, in Umfangsrichtung unterteilte Ortsselektivität zur ortsselektiven Erfassung neuronaler elektrischer Signale aus dem zu untersuchenden Nervenfaserbündel NFB. Die jeweils axial beidseitig neben den drei ersten Elektrodenstrukturen 3 angeordneten ersten Elektrodenstreifen 5, die das Nervenfaserbündel NFB vollständig ringförmig umfassen, dienen im Falle der ortsselektiven Erfassung neuronaler elektrischer Signale als Massepotential; gilt es hingegen selektiv ausgewählte Nervenfasern NF innerhalb des Nervenfaserbündels NFB elektrisch zu stimulieren, so dienen diese ersten Elektrodenstreifen 5 jeweils als Anode bzw. als Gegenpolarität.

Die Dreifach- bzw. Tripol-Anordnung der jeweils ersten Elektrodenstrukturen 3, über deren jeweils erste Elektrodenflächen 4 monopolar neuronale elektrische Signale erfasst bzw. elektrische Signale zu Zwecken der ortsselektiven Stimulation abgegeben werden können, ermöglicht es Impedanzänderungen aufgrund von Gewebewachstum an den metallischen Elektrodenflächen 4 festzustellen und auswertetechnisch zu eliminieren, zum anderen können blutdruckrelevante neuronale Signale, die mit einem leichten zeitlichen Versatz durch die Tripolanordnung axial längs einer entsprechenden Nervenfaser NF laufen, mittels geeigneter tripolarer Verstärkung detektiert werden. Neben den vorstehend bezeichneten ersten Elektrodenstrukturen 3 sowie ersten, jeweils eine Ringform annehmenden Elektrodenstreifen 5, die allesamt an der in Figur 2a der Zeichenebene zugewandten Trägersubstratoberfläche 1' aufgebracht sind und über entsprechend elektrische Leitbahnen L proximalseitig an Verbindungsstrukturen V enden, befindet sich rückseitig am Trägersubstrat 1 eine zweite Elektrodenanordnung in Form von Referenzelektroden 12, die zum einen zur Erfassung des intrakorporalen elektrischen Hintergrundmassensignals bzw. Rauschniveaus dienen, das der Signalauswertung zugrunde gelegt wird, zum anderen die Möglichkeit eröffnen, EKG-Signale mit Hilfe der Manschettenelektrode E zu erfassen. Die als Manschettenelektrode E implantierbare Elektrodenanordnung ist über die elektrischen Verbindungsstrukturen V mit einem hermetisch gekapselten Signaldetektor und -generator 6 verbindbar, der ebenfalls als Implantat ausgebildet ist.

Mit der bekannten implantierbaren Elektrodenanordnung konnte im Rahmen von Tierversuchen an Ratten gezeigt werden, dass mit Hilfe der gleichverteilt um das Nervenfaserbündel NFB tripolar angeordneten, insgesamt 24 ersten Elektrodenflächen Blutdruck korrelierte, neuronale elektrische Zeitsignale, im Folgenden als barorezeptive Signale bezeichnet, erfasst werden können, die darüber hinaus hinsichtlich ihrer in Umfangsrichtung abhängigen Signalpegel zur Ortung der barorezeptiven Nervenfasern dienen. Die Stimulation erfolgte tripolar jeweils mit jener Elektrodenfläche 4 oder jenen Elektrodenflächen 4 der mittig angeordneten ersten Elektrodenstruktur 3 der Tripol-Anordnung, über die bei der Detektion der jeweils größte Signalpegel unter den barorezeptiven Signalen erfasst wurde. Es konnte gezeigt werden, dass durch selektive Stimulation barorezeptiver Nervenfasern der Blutdruck zuverlässig deutlich gesenkt werden kann, wobei sich lediglich eine sehr schwache Bradykardie (Pulsreduzierung unter 60 Schläge pro Minute) sowie eine kaum nennenswerte Bradypnoe (Reduzierung der Atmung unter 20 Atemzüge pro Minute) einstellten.

Zur selektiven elektrischen Stimulation der barorezeptiven Nervenfasern wurden elektrische Stimulationssignale unter Zugrundelegung einer bestimmten Kombination an fest vorgegebenen Stimulationsparametern an die jeweils selektierten Elektrodenflächen 4 der mittig angeordneten Elektrodenstruktur appliziert. Hierbei wurden die Stimulationssignale in Form von elektrischen Stimulationsereignissen in frei wählbaren Intervallen an die selektierten Nervenfasern appliziert, bspw. wurde alle 20 Sekunde ein aus 100 Einzelpulsen zusammengesetzter elektrischer Stimulus über die jeweils selektierte(n) Elektrodenfläche(n) an das Nervenfaserbündel appliziert. Jeder Einzelpuls besaß dabei eine Stimulationspulsdauer von 0,6 ms mit einer anodischen und kathodischen Stimulationsamplitude von jeweils 0,8 mA, wodurch eine Elektrodenpolarisierung ermöglicht wird. Mit einer Wiederholrate der Einzelpulse, der sogenannten Stimulationsfrequenz, von 40 Hz betrug die Gesamtdauer eines einzelnen, elektrischen Stimulus, 100 x 25 ms, somit 2,5 Sekunden. Bei den durchgeführten Stimulationsversuchen an Ratten wurden unterschiedliche, jeweils fest vorgegebene Stimulationsparameter verwendet, nämlich jeweils eine Stimulationsfrequenz von 30 bis 50 Hz, eine Stimulationspulsdauer von 0,1 ms bis 0,5 ms sowie eine Stimulationsamplitude von 0,3 mA bis 1,5 mA.

Gleichwohl die im Rahmen der bisherigen Tierversuche gewonnenen Erkenntnisse in Hinblick auf die Beeinflussung des Blutdruckes durch selektive elektrische Stimulation barorezeptiver Nervenfasern vielversprechend erscheinen, sind zumindest die quantitativen Zusammenhänge zwischen dem elektrischen Stimulationsereignis und der biologischen Antwort in Form einer auf der Grundlage organischer Regelungsmechanismen initiierte Blutdrucksenkung noch weitgehend unverstanden. Insbesondere bei größeren Tieren, als die bisher in den Tierversuchen eingesetzten Ratten, oder gar beim Menschen, gilt es mit einem größeren Maß an Vorbestimmtheit regulative Stimulationen vorzunehmen, deren organisch herbeigeführtes Regelungsergebnis in einem quantitativ determinierten Toleranzbereich zu liegen kommen soll.

### Darstellung der Erfindung

Der Erfindung liegt die Aufgabe zugrunde eine implantierbare Anordnung zur ortsselektiven Erfassung neuronaler elektrischer Signale, die sich längs wenigstens einer in einem Nervenfaserbündel enthaltenen Nervenfasern ausbreiten, sowie zur selektiven elektrischen Stimulation wenigstens einer Nervenfaser mit einer vorstehend erläuterten Elektrodenanordnung derart weiterzubilden, so dass die stimulative Einflussnahme auf bestimmte Bereiche des vegetativen Nervensystems, insbesondere auf den Vagusnerv zu Zwecken der Blutdruckbeeinflussung mit einer signifikant größeren Präzision vorgenommen werden kann. Gilt es entsprechende regulative Maßnahmen an größeren Lebewesen als Ratten, vor allem an Menschen, vorzunehmen, so muss sichergestellt sein, dass sich der angestrebte neuronale, physiologische und/oder organische Zustand zumindest innerhalb eines absehbar quantifizierbaren Toleranzbereiches einstellt. Sämtliche hierzu erforderlichen Maßnahmen sollen zudem unerwünschte biologische Nebenwirkungen ausschließen. Grundsätzlich soll die Anordnung neben der angestrebten Blutdruckbeeinflussung alternativ oder in Kombination auch für beliebig andere vegetative aber auch sensomotorische Zustandsgrößen zum Zwecke einer zielgerichteten Einflussnahme fähig sein.

Die Lösung der der Erfindung zugrunde liegenden Aufgabe ist im Anspruch 1 angegeben. Den Lösungsgedanken in vorteilhafter Weiser weiterbildende Merkmale sind Gegenstand der Unteransprüche sowie der weiteren Beschreibung unter Bezugnahme auf die Ausführungsbeispiele zu entnehmen.

In Abkehr zur vorstehend erläuterten Vorgehensweise zur elektrischen Stimulation wenigstens einer selektierten Nervenfaser mit jeweils starr vorgegebenen Stimulationsparametern, d.h. Stimulationspulsdauer, Stimulationsamplitude und Stimulationsfrequenz, ermöglicht die lösungsgemäß ausgebildete implantierbare Anordnung die Erzeugung von elektrischen Stimulationssignalen, die in ihrer Form, d. h. dem zeitlichen Amplitudenverlauf, der Pulswellenform natürlicher Blutdrucksignale weitgehend angeglichen werden sowie auch in zeitlicher Überlagerung mit den natürlichen Blutdrucksignalen an die barorezeptiven Nervenfasern appliziert werden, so dass die sich längs der barorezeptiven Nervenfasern von den Barorezeptoren hin zum Hirnstamm übertragenen, natürlichen Blutdrucksignale regelrecht überschrieben werden. Somit erfolgt die lösungsgemäße Stimulation nicht, wie bisher, aus einem pulsativen "an-aus-an-aus"-Schema, sondern die längs der barorezeptiven Nervenfasern applizierten technischen, elektrischen Stimulationssignale werden dem Hirnstamm mit einer an den natürlichen Signalrhythmus angepassten Stimulationsfrequenz zugeleitet, d. h. jeweils in jenen natürlichen Zeitfenstern, in denen der Hirnstamm die natürlichen Blutdrucksignale erwartet.

Die natürliche Form einer Pulswelle, die durch die barorezeptiven Felder im Aortenbogen läuft, besitzt typischerweise eine Pulsdauer von weniger als eine Sekunde und ist darüber hinaus durch einen starken, schnellen und nicht linearen Pulswellenanstieg und einen darauf folgenden langsamen, ebenfalls nicht linearen Abfall charakterisiert. Diese mechanische Pulswelle wird von den Barorezeptoren in eine neuronale, elektrische Signalform transduziert. Dieses neuronal, elektrische Signal wird über den Vagus an den Hirnstamm geleitet und enthält die Informationen bezüglich der Stärke und Dauer der mechanischen Pulswelle. In Anpassung an diese natürliche neuronale elektrische Signalform erfolgt die vermittels der implantierbaren Anordnung technisch initiierte Einflussnahme auf den natürlichen organischen Regelmechanismus durch ein zeitlich kohärentes Überschreiben der natürlichen, neuronalen, elektrischen Zeitsignale mit technischen, an die wenigstens eine selektierte, barorezeptive Nervenfaser applizierten, elektrischen Stimulationssignalen, deren Amplitudenpegel gegenüber den natürlichen, neuronalen, elektrischen Zeitsignalen angehoben oder abgesenkt sind, je nach angestrebten Therapieziel. Auf diese Weise wird der natürliche, organische Blutdruckregelmechanismus nicht oder nicht signifikant irritiert, d. h. der Hirnstamm, der die technisch manipulierten elektrischen Stimulationssignale empfängt, vermag keinen Unterschied zu den natürlichen, neuronalen, elektrischen Zeitsignalen zu erkennen. In Folge dessen wird der natürliche organische Regelmechanismus aktiviert, der auf ganz natürliche Weise zu einem Regelungsergebnis führt, das sich in Form einer bestimmten, zu erwartenden Blutdruckwerteinstellung auszeichnet.

Ferner bietet die lösungsgemäße implantierbare Anordnung die Möglichkeit einer autonom kontrollierenden Blutdruckwertüberwachung, d. h. der natürliche organische Regelmechanismus wird nur in jenen Fällen aktiviert, in denen eine signifikante Abweichung von einem Blutdrucknormwert festgestellt wird. In weiterer vorteilhafter Weise ist es möglich, die implantierbare Anordnung durch Selbstkontrolle, d. h. in Art einer closed-loop-Funktion zu betreiben, bei der das aufgrund einer elektrischen Stimulation hervorgerufene organische Regelergebnis erfasst, bewertet, und falls erforderlich, einer entsprechenden Nachregelung unterworfen wird.

Hierzu zeichnet sich die lösungsgemäße implantierbare Anordnung zur ortsselektiven Erfassung neuronaler elektrischer Signale, die sich längs wenigstens einer in einem Nervenfaserbündel enthaltenden Nervenfaser, vorzugsweise einer barorezeptiven Faser, ausbreiten, sowie zur selektiven elektrischen Stimulation der wenigstens einen Nervenfaser durch folgende Komponenten aus:
Im Weiteren wird ohne Einschränkung des allgemeinen Erfindungsgedankens die lösungsgemäße Anordnung am Beispiel der Beeinflussung des Blutdrucks als physiologischer Parameter erläutert. Selbstverständlich kann die implantierbare Anordnung auch zur Beeinflussung anderer physiologischer Parameter, bspw. Atemfrequenz, Herzschlagrate, Körpertemperatur etc. oder bei anderen Krankheitsbildern eingesetzt werden, bspw. bei Autoimmunkrankheiten, Herzrhythmusstörungen, schweren Depression, Epilepsie etc. Die implantierbare Anordnung lässt sich für die Therapierung alternativer Körperfunktionen ebenso auch an anderen peripheren Nerven oder Nerven des zentralen oder vegetativen Nervensystems einsetzen. Als ein Beispiel sei der Bereich motorischer Neuroprothesen nach zentraler Lähmung bei Rückenmarks- oder Gehirnverletzungen genannt. In diesen Fällen können die sensorischen Signale der Druck- und Lagerezeptoren beispielsweise der Hand selektiv mit implantierbaren Anordnung aufgenommen werden und die Griffkraft entsprechend einer Sollwertvorgabe selbsttätig geregelt werden. Im Bereich der Neuromodulation kann auch eine Rehabilitation nach Schlaganfall und Hemiparese mit Hilfe der implantierbaren Anordnung in Aussicht gestellt werden. Hierbei kann das sensorische Signal verstärkt eingekoppelt werden um den Rehabilitationserfolg zu verbessern. Denkbar ist auch ein geregelter Atemstimulator mit Hilfe der implantierbaren Anordnung über den Nervus phrenicus am Zwerchfell, die Modulation des sympathischen Nervensystems am Grenzstrang oder die effiziente Schmerztherapie durch hochselektive periphere Nervenstimulation.

Zur ortsselektiven Erfassung neuronaler elektrischer Signale längs ausgewählter Nervenfasern innerhalb eines Nervenfaserbündels sowie auch zur selektiven elektrischen Stimulation der wenigstens einen selektierten Nervenfaser ist eine implantierbare Elektrodenanordnung vorgesehen, die auf einem um das Nervenfaserbündel manschettenartig anlegbaren biokompatiblen Trägersubstrat aufgebracht ist, das eine im implantierten Zustand dem Nervenfaserbündel zugewandt orientierte, geradzylinderförmige Trägersubstratoberfläche aufweist. Zudem ist an dem biokompatiblen Trägersubstrat eine zweite Elektrodenanordnung zur Erfassung des EKG-Signals, das die Herzaktivität repräsentiert, angeordnet. Nicht notwendigerweise muss die zweite Elektrodenanordnung auf der gleichen Trägersubstratoberfläche des Trägersubstrats aufgebracht sein, wie die vorstehend erste Elektrodenanordnung.

Die implantierbare Elektrodenanordnung, d. h. wenigstens umfassend die erste und zweite Elektrodenanordnung, ist mit einer Auswerte-/Steuereinheit elektrisch verbunden oder mit dieser verbindbar ausgebildet, in der die ortsselektiv erfassten, neuronalen, elektrischen Signale sowie auch das EKG-Signal zeitaufgelöst auswertbar sind, so dass ein zum Blutdruck, korreliertes neuronales Zeitsignal ableitbar ist. Die als Digitaler Signalprozessor oder Microcontroller ausgebildete Auswerte-/Steuereinheit vermag Signaldaten zu verarbeiten sowie Steuersignale zu generieren.

Eine mit der Auswerte-/Steuereinheit verbundene erste Komparatoreinheit dient der Ermittlung eines charakteristischen relativen Zeitversatzes zwischen dem messtechnisch erfassten EKG-Signals und dem mit dem Blutdruck korrelierten neuronalen Zeitsignal.

In vorteilhafter Weise wird hierbei der zeitliche Abstand zwischen der R-Zacke des EKG-Zeitsignals und einem Signalflankenpunkt längs der sich positiv stark aufsteigenden Signalflanke des mit der Blutdruckwelle korrelierten messtechnisch erfassten Zeitsignals ermittelt. Das mit der Pulswelle bzw. dem Blutdruck korrelierte, messtechnisch erfasste, neuronale Zeitsignal zeichnet sich durch eine von der Ausgestaltung der ersten Elektrodenanordnung abhängigen, zumeist mehrphasigen Signalform aus, der ein charakteristischer Signalflankenpunkt zuordenbar ist, der zur Ermittlung eines Zeitversatzes zum zeitlich unmittelbar vorauseilenden EKG-Signals dient. Der ermittelte Zeitversatz zwischen dem EKG-Signal und der Blutdruckwelle bzw. der Pulswelle bzw. dem mit der Blutdruckwelle korrelierten messtechnisch erfassten Zeitsignals dient im Weiteren zu einer zeitlich exakten Anpassung der Erzeugung von technischen Stimulationssignalen an die sich längs der barorezeptiven Nervenfasern ausbreitenden, natürlichen, neuronale elektrischen Signale.

Ferner ermittelt die Auswerte-/Steuereinheit ein Zeitfenster, innerhalb dem das Blutdruck korrelierte neuronale Zeitsignale über einen bestimmten Amplitudenpegel liegt, d.h. das Zeitfenster entspricht der Pulsdauer einer Blutdruckwelle. Ziel ist es zu Zwecken der Blutdruckbeeinflussung innerhalb dieses mit Hilfe der Auswerte-/Steuereinheit ermittelten Zeitfensters an die wenigstens eine selektierte barorezeptive Nervenfaser ein technisches, elektrisches Stimulationssignal zu applizieren, so dass das Gehirn das elektrische Stimulationssignal mit einer an die natürliche Pulswellendauer angepasste Signaldauer und zu einem Zeitpunkt erhält, zu dem das Gehirn die normalen, d. h. natürlichen Blutdrucksignale erwartet.

Ferner ist die Auswerte-/Steuereinheit mit einem ersten Funktionsgenerator elektrisch verbunden, der innerhalb jeweils des durch die Auswerte-/Steuereinheit ermittelten Zeitfensters, das gegenüber dem EKG-Signal den ermittelten relativen Zeitversatz aufweist, ein aus einer Vielzahl n Einzelpulsen zusammensetzendes elektrisches Stimulationssignal generiert, dessen Phase und zeitlicher Amplitudenverlauf an Phase und Amplitudenverlauf des abgeleiteten, mit dem physiologischen Parameter, vorzugsweise Blutdruck, korrelierte, neuronale Zeitsignal angepasst ist. Vorzugsweise unterscheidet sich das elektrische Stimulationssignal lediglich durch den sich zeitlich variierenden Amplitudenpegel, der im Falle einer Bluthochdrucktherapierung größer bzw. höher gewählt ist als jener des mit dem natürlichen Blutdruck korrelierten, neuronalen Zeitsignals. Somit erhält das Gehirn die Information über einen stark erhöhten Blutdruck, gegen den entsprechende natürliche organische Regelungsmechanismen aktiviert werden.

Zur Umwandlung und Weiterleitung der sich aus der Vielzahl n Einzelpulsen zusammensetzenden elektrischen Stimulationssignals in Form eines Stromsignals sind der erste Funktionsgenerator sowie auch die erste Elektrodenanordnung der implantierbaren Elektrodenanordnung mittel- oder unmittelbar über einen ersten Signal-Strom-Wandler verbunden, der das elektrische Stimulationssignal zur selektiven elektrischen Stimulation der wenigstens einen Nervenfaser an die erste Elektrodenanordnung leitet.

Mit Ausnahme der implantierbaren Elektrodenanordnung, deren erste Elektrodenanordnung in körperlichem, d. h. elektrischem Kontakt mit dem Epineurium des Nervenfaserbündels tritt, sind alle übrigen Komponenten der implantierbaren Anordnung, d. h. die Auswerte-/Steuereinheit, die erste Komperatoreinheit, der erste Funktionsgenerator sowie auch der erste Signal-Strom-Wandler in einem implantierbaren Modul integriert, d. h. von einer aus biokompatiblen Material bestehenden Kapsel fluiddicht umgeben, wobei zur elektrischen Kontaktierung der innerhalb des implantierbaren Moduls zusammengefasste Komponenten mit der implantierbaren Elektrodenanordnung wenigstens eine elektrische Verbindungsstruktur vorgesehen ist.

Durch die zeitlich kohärente Übereinstimmung der selektiven elektrischen Stimulation mit der Übertragung natürlicher, neuronaler elektrischer Signale längs selektierter barorezeptiver Nervenfasern, sowie die Anpassung der Stimulationssignale an Signaldauer und Signalform an die natürlichen barorezeptiven neuronalen Signale spiegelt sich der Unterschied lediglich in einem gegenüber den natürlichen barorezeptiven Signalen zumeist angehobenen, d. h. höheren, sich zeitlich variierenden Amplitudenpegel wieder. Selbstverständlich ist es auch möglich geringere Amplitudenpegel mit Hilfe der lösungsgemäßen implantierbaren Anordnung an die wenigstens eine selektierte Nervenfaser zu applizieren. Um das Maß der technischen Amplitudenpegelüberhöhung bzw. -reduzierung quantitativ festzulegen, sieht die implantierbare Anordnung wenigstens eine zweite Komperatoreinheit vor, die mit der Auswerte-/Steuereinheit elektrisch verbunden ist, die wenigstens einen Signalpegel, der dem mit dem Blutdruck korrelierten, neuronalen Zeitsignal zugeordnet ist, mit wenigstens einem Referenzsignal vergleicht und in Folge dessen einen Differenzpegelwert generiert. Die Auswerte-/Steuereinheit legt im Weiteren zumindest auf der Grundlage des ermittelten Differenzpegelwertes wenigsten den zeitlichen Amplitudenverlauf des Stimulationssignals fest, d. h. weicht das mit Hilfe der implantierbaren Anordnung messtechnisch detektierte, mit dem Blutdruck korrelierte neuronale Zeitsignal signifikant von dem vorgegebenen Referenzsignal ab, so wird je nach Regelungsbedarf der sich zeitlich variierende Amplitudenpegel des elektrischen Stimulationssignal gegenüber dem messtechnisch erfassten, mit dem Blutdruck korrelierten neuronalen Zeitsignals angehoben bzw. abgesenkt. Im Falle einer Bluthochdrucktherapierung gilt es zumeist den zeitlich variierenden Amplitudenpegel signifikant anzuheben, um auf diese Weise dem Gehirn die Information über einen erhöhten Blutdruck zuzuführen, das im Weiteren im Rahmen natürlicher organischer bzw. biologischer Regelungsmechanismen den überhöht festgestellten Blutdruckpegel zu reduzieren versucht.

Die vorstehend erläuterte elektrische Stimulation, die mit Hilfe der in Figur 2 a und b illustrierten Manschettenelektrodenanordnung vorgenommen wird, erfolgt längs der barorezeptiven Nervenfasern mit isotroper Signalrichtungseinkopplung, d. h. ohne Vorgabe einer festgelegten Signalausbreitungsrichtung, so dass sich die elektrischen Stimulationssignale sowohl längs afferenter sowie auch efferenter Nervenfasern ausbreiten können. Um die Signalausbreitung von elektrischen Stimulationssignalen längs efferenter Nervenfasern, d.h. zum Herzen gerichtet, zu unterbinden, ohne dabei einen signifikant nachhaltigen Einfluss auf nicht barorezeptive, afferente sowie efferente Nervenfasern innerhalb des Nervenfaserbündels auszuüben, eignet sich eine gegenüber der in Figur 2 beschriebenen Elektrodenanordnung modifizierte Manschettenelektrodenanordnung, die wenigstens eine zur Inhibition einer unidirektionalen elektrischen Signalübertragung längs wenigstens einer selektierten Nervenfaser innerhalb eines Nervenfaserbündels ausgebildeten dritten Elektrodenanordnung ergänzt ist.

Die ebenso auf dem gleichen, einstückig zusammenhängend ausgebildeten Trägersubstrat an der gleichen Trägersubstratoberfläche, wie die erste Elektrodenanordnung aufgebrachte dritte Elektrodenanordnung steht in einer räumlich festen Zuordnung zur ersten Elektrodenanordnung, insbesondere zu den ersten Elektrodenflächen der wenigstens drei ersten Elektrodenstrukturen, mit deren Hilfe barorezeptive Nervenfasern innerhalb des Nervenfaserbündels ortsselektiv erfasst und darüber hinaus selektiv elektrisch stimulierbar sind. In Kenntnis der lokalisierten barorezeptiven Nervenfasern lässt sich die dritte Elektrodenanordnung zu Zwecken einer selektiven Inhibition der barorezeptiven Nervenfasern zur Unterdrückung einer Weiterleitung elektrischer Stimulationssignale längs efferenter, d.h. zum Herzen führender, Nervenfasern nutzen. Hierzu dienen wenigstens zwei, vorzugsweise vier oder mehr zweite Elektrodenflächen wenigstens einer dritten Elektrodenstruktur, die gleichfalls wie die ersten Elektrodenflächen einer der wenigstens drei ersten Elektrodenstrukturen in Umfangsrichtung der dem Nervenfaserbündel zugewandt orientierten, sich geradzylinderförmig ausbildenden Trägersubstratoberfläche gleich verteilt angeordnet sind. Zu Zwecken der Inhibition lokalisierter efferenter barorezeptiver Nervenfasern wird wenigstens eine der dritten Elektrodenflächen der dritten Elektrodenstruktur elektrisch aktiviert, wodurch es zu einer gezielt, zeitlich begrenzten, selektiven Inhibition der betreffenden efferenten Nervenfaser kommt. Hierbei tritt von der jeweils wenigstens einen aktivierten, dritten Elektrodenfläche ein elektrisches Polarisationsfeld in das Nervenfaserbündel ein und wechselwirkt vornehmlich mit der zu inhibieren Nervenfaser. Um das sich während der Inhibition in das Nervenfaserbündel ausbreitende elektrische Polarisationsfeld axial zu begrenzen, dienen jeweils zur dritten Elektrodenstruktur axial beidseitig angebrachte zweite Elektrodenstreifen, die im implantierten Zustand der Manschettenelektrode das Nervenfaserbündel vollständig umschließende Ringelektroden darstellen.

Zu Zwecken der Inhibition ausgewählt efferenter Nervenfasern ist die modifizierte implantierbare Elektrodenanordnung derart an das Nervenfaserbündel zu applizieren, so dass die zusätzlich vorgesehene dritte Elektrodenanordnung dem Herzen, bzw. den barorezeptiven Rezeptoren zugewandt, d.h. caudal, und die erste Elektrodenanordnung, mit der die selektive Erfassung neuronaler elektrischer Signale sowie auch die elektrische Stimulation lokalisierter Nervenfasern vorgenommen wird, dem Gehirn, d.h. rostral, längs des Nervenfaserbündels zugewandt orientiert sind.

Mit Hilfe der dritten Elektrodenanordnung lässt sich die Inhibition entweder im Wege eines sog. anodalen Blockes oder durch Applikation von sinusförmigen Signalen von Frequenzen im Kilohertzbereich realisieren. Im Falle des anodalen Blocks wird wenigstens eine der zweiten Elektrodenflächen anodisch polarisiert, wodurch ein am Ort der efferenten Nervenfaser vorherrschendes Spannungspotential erzeugt wird, durch das eine aktivierende Stimulation der entsprechenden Nervenfaser unterdrückt wird. Ebenso kann eine Inhibition im Wege einer Hochfrequenzsignalapplikation erzielt werden, bei der an wenigstens einer ausgewählten dritten Elektrodenfläche ein hochfrequentes elektrisches Inhibitionssignal angelegt wird, wodurch die elektrischen Signalübertragungsmechanismen längs der efferenten Nervenfasern kurzzeitig zum Erliegen kommen.

In beiden Fällen wirkt die lösungsgemäß vorgesehene dritte Elektrodenanordnung aufgrund ihrer räumlich axialen Begrenztheit, die durch den axialen Abstand beider dritten Elektrodenstreifen gegeben ist, trotz ihrer räumlichen Nähe zur ersten Elektrodenstruktur, immerhin sollte die implantierbare Elektrodenanordnung eine axiale Länge von 4 cm nicht überschreiten, axial räumlich begrenzt längs der zu inhibierenden efferenten Nervenfasern, so dass die gehirnseitig längs des Nervenfaserbündels angeordnete erste Elektrodenanordnung in den jeweils lokalisierten afferenten Nervenfasern zum Gehirn führende elektrische Stimulationssignale unbeeinflusst von dem Inhibitionsmechanismus einkoppeln kann. Auf diese Weise können jegliche Nebenwirkungen, bedingt durch mögliche direkte Stimulationen in Richtung der zum Herz führenden, d.h. efferenten Nervenfasern ausgeschlossen werden.

In vorteilhafter Weise sind die dritten Elektrodenflächen der dritten Elektrodenstruktur im implantierten Zustand der Manschettenelektrode längs einer virtuellen Kreislinie gleich verteilt angeordnet, um auf diese Weise relativ zum Umfangsrand eines Nervenfaserbündels lokalisierte efferente Nervenfasern selektiv und effektiv zu inhibieren.

Nicht notwendigerweise jedoch in vorteilhafter Form sind die dritten Elektrodenflächen in Form und Größe identisch untereinander ausgebildet, wobei ihre axialen Erstreckungen jeweils identisch gewählt sind, gleichsam den axialen Erstreckungen der ersten Elektrodenflächen der ersten drei Elektrodenstrukturen. Die in Umfangsrichtung orientierte Erstreckung der jeweils dritten Elektrodenflächen ist größer gewählt als die in Umfangsrichtung orientierte Erstreckung der ersten Elektrodenflächen. Somit weisen die dritten Elektrodenflächen gegenüber den ersten Elektrodenflächen vorzugsweise ein größeres Flächenmaß auf, wodurch die Ortsselektivität, mit der die dritten Elektrodenflächen bestimmte efferente Nervenfasern elektrisch zu polarisieren vermögen geringer ist als die Ortsselektivität, mit der die ersten Elektrodenflächen lokalisierte Nervenfasern elektrisch zu stimulieren vermögen. Alternativ können die dritten Elektrodenflächen anstelle einer Rechtecksform auch als Kreisflächen ausgebildet sein. Dies hat den Vorteil, dass sich keine lokalen, durch Kanten oder Ecken bedingten elektrische Potenzialfeldspitzen ausbilden.

Die dritte Elektrodenanordnung ist vorzugsweise in Form einer Tripolar-Elektrodenanordnung ausgebildet, d. h. die dritte Elektrodenstruktur ist axial beidseitig von jeweils einem ringförmig ausgebildeten dritten Elektrodenstreifen begrenzt, wobei der axiale Abstand zwischen beiden dritten Elektrodenstreifen längs des Trägersubstrates vorzugsweise zwischen 0,5 cm und 3 cm, insbesondere zwischen 0,75 cm und 1,25 cm gewählt ist. Die ringförmig ausgebildeten dritten Elektrodenstreifen besitzen vorzugsweise eine axiale Erstreckung zwischen 1 µm und 5 mm, vorzugsweise zwischen 100 µm und 4000 µm.

Die dritten Elektrodenflächen der dritten Elektrodenstruktur sind axial mittig zwischen beiden dritten Elektrodenstreifen angeordnet und verfügen über eine axiale Erstreckung, so dass der jeweils axiale Abstand zu den zweiten Elektrodenstreifen größer ist als ihre eigene axiale Erstreckung.

Insbesondere im Hinblick auf die Möglichkeit der Durchführung von depolarisierenden Maßnahmen ist es denkbar, anstelle einer dritten Elektrodenstruktur drei axial beabstandete dritte Elektrodenstrukturen zwischen den dritten Elektrodenstreifen anzuordnen, gleichsam zur Ausbildung der jeweils ersten Elektrodenstruktur innerhalb der ersten Elektrodenanordnung. Nur der Vollständigkeit halber sei erwähnt, dass es ebenso denkbar wäre, auch mehr als drei erste und dritte Elektrodenstrukturen zwischen den jeweiligen ersten und dritten Elektrodenstreifen anzuordnen. So könnten drei, fünf, sieben oder mehr ungeradzahlige erste und/oder dritte Elektrodenstrukturen vorgesehen werden.

In einem bevorzugten Ausführungsbeispiel umfasst eine dritte Elektrodenstruktur vier dritte Elektrodenflächen, deren Elektrodenflächenmaß jeweils kleiner als ein Viertel der Flächengröße jeweils eines dritten Elektrodenstreifens gewählt ist. Da die sowohl in der ersten als auch in der dritten Elektrodenanordnung vorgesehenen ersten bzw. dritten Elektrodenstreifen jeweils als Masse- oder Gegenpole zur Polarisierung der jeweils ersten bzw. dritten Elektrodenstruktur dienen, sind aus Gründen ladungssymmetrischer Verhältnisse die Flächengrößen der ersten und dritten Elektrodenstreifen jeweils identisch gewählt. Vorstellbar ist jedoch auch eine individuell unabhängige Flächengrößenwahl in der Ausbildung der ersten und dritten Elektrodenstreifen.

Ferner hat es sich als vorteilhaft erwiesen, sämtliche Elektroden der dritten Elektrodenanordnung, d.h. die dritten Elektrodenflächen und dritten Elektrodenstreifen, aus einem elektrisch leitenden Material zu fertigen, das über eine geringere Ladungsübertragungskapazität verfügt als das Elektrodenmaterial, aus dem die ersten Elektrodenflächen der ersten Elektrodenanordnung bestehen. Als besonders geeignetes Material mit einer besonders hohen Ladungsübertragungskapazität wird Iridium-Oxid zur Ausbildung der jeweils ersten Elektrodenflächen der ersten Elektrodenanordnung verwendet, wohingegen das Material der dritten Elektrodenflächen und dritten Elektrodenstreifen aus Platin oder aus einem elektrisch leitfähigen Polymer besteht.

Sämtliche Elektrodenflächen sowohl der ersten als auch dritten Elektrodenanordnung sind vorzugsweise bündig zur Trägersubstratoberfläche des Trägersubstrats ausgebildet oder gegenüber dieser abgesetzt angeordnet, so dass sie die Trägersubstratoberfläche nicht überragen, um einen möglichst schonenden Oberflächenkontakt zum Epineurium des Nervenfaserbündels herzustellen. Durch den nichtinvasiven Oberflächenkontakt kann die implantierbare Elektrodenanordnung längs des Nervenfaserbündels operativ leicht appliziert und positioniert werden, wobei das Epineurium nur minimal bis nicht irritiert wird.

Um ferner implantationsbedingten Gewebeirritationen und entzündlichen Reaktionen entgegen zu treten, bietet es sich an, das aus einem biokompatiblen Polymer bestehende Trägersubstrat zumindest in jenen Bereichen, die mit dem Nervenfaserbündel in unmittelbaren Oberflächenkontakt treten, mit einem Inflammationsreaktionen inhibierenden Wirkstoff zu versehen.
Eine weitere Maßnahme zur Reduzierung mechanischer Reizungen des Nervenfaserbündels, die durch den Oberflächenkontakt mit der manschettenartigen Manschettenelektrode entstehen können, betrifft eine Abrundung axialer Begrenzungskanten der das Nervenfaserbündel umschließenden Trägersubstrats derart, dass das biokompatible Trägersubstrat im Bereich der dem Nervenfaserbündel zugewandt orientierten, geradzylinderförmigen Trägersubstratoberfläche jeweils axial sich gegenüberliegende Randbereiche aufweist, an denen das Trägersubstrat über eine größere Substratdicke verfügt als im übrigen Trägersubstratbereich, wobei die Randbereiche über abgerundete Randkanten verfügen.

Im Bereich der dritten Elektrodenanordnung, die zur elektrischen Inhibition lokalisierter Nervenfasern dient, sieht eine weitere bevorzugte Ausführungsform wenigstens eine, vorzugsweise mehrere Lichtwellenleiteröffnungen bzw. -aperturen vor, über die Licht durch das Epineurium des Nervenfaserbündel appliziert bzw. eingekoppelt werden kann. Die Lichtwellenleiteröffnungen sind vorzugsweise axial zu beiden zweiten Elektrodenstreifen benachbart angeordnet und in Form, Größe und Verteilung entsprechend den dritten Elektrodenflächen der dritten Elektrodenstruktur nachgebildet. Durch Vorsehen mehrerer, räumlich separierter Lichtwellenleiter, die an der Trägersubstratoberfläche dem Nervenfaserbündel zugewandt münden, können dem Nervenfaserbündel einheitliche oder unterschiedliche optische Signale mit unterschiedlichen Wellenlängen zu Zwecken einer optischen Aktivierung neuronaler optogenetischer Reaktionen innerhalb des Nervenfaserbündels appliziert werden. So lassen sich durch eine Vielzahl geeignet angeordneter Lichtwellenaustrittsöffnungen bzw. -aperturen innerhalb des Nervenfaserbündels neuronale Aktivierungs- oder Inhibitionsreaktionen ortsselektiv auslösen, die alternativ oder in Ergänzung zu den über die Elektrodenflächen hervorgerufenen neuronalen Prozessen vorgenommen werden können.

Wie bereits erwähnt, gilt es die lösungsgemäß ausgebildete implantierbare Elektrodenanordnung derart längs des Nervenfaserbündels zu applizieren, so dass die dritte Elektrodenanordnung längs des Nervenfaserbündels in Richtung zum Herzen zugewandt zu liegen kommt. Auf diese Weise ist sichergestellt, dass efferente Nervenfasern inhibiert werden können, wohingegen die dem Gehirn längs des Nervenfaserbündels zugewandt orientierte erste Elektrodenanordnung zu Zwecken der selektiven Stimulation lokalisierter afferenter, d. h. zum Hirn führenden Nervenfasern eingesetzt werden kann. Sollte es einen Bedarf geben, afferente Nervenfasern selektiv zu inhibieren, so kann die modifizierte implantierbare Elektrodenanordnung mit umgekehrter Orientierung längs des Nervenfaserbündels implantiert werden. Eine weitere mögliche Ausführungsform sieht eine zweite inhibierende dritte Elektrodenanordnung vor, die axial neben der ersten Elektrodenanordnung, der dritten Elektrodenanordnung gegenüberliegend angebracht ist.

Die intrakorporale Implantation der das Nervenfaserbündel manschettenartig umgebenden Elektrodenanordnung ist ferner mit dem grundsätzlichen Problem konfrontiert, dass die auf dem Polyimid-Trägersubstrat aufgebrachten Elektrodenstreifen und Elektrodenflächen einem andauernden feuchten Milieu ausgesetzt sind, wodurch Degradationserscheinungen insbesondere an den flächigen Verbindungen zwischen den Elektrodenflächen und dem Polyimid-Trägersubstrat auftreten können, die zu lokalen Ablösungen und damit verbunden zumindest zu Kontaktdegradierungen führen, durch die letztlich die elektrische Effizienz der Elektrodenanordnung beeinträchtigt wird. Um diesen Milieu bedingten Ablösungserscheinungen zwischen metallischen Elektrodenflächen und dem Polyimid-Trägersubstrat zu begegnen, verfügen in einer bevorzugten Ausführungsform zumindest die ersten und dritten Elektrodenstreifen jeweils über wenigstens eine lokale Öffnung, wobei die ersten und dritten Elektrodenstreifen derart mit dem Trägersubstrat bzw. der Trägersubstratoberfläche flächig verbunden sind, so dass das Polymer bzw. Polyimid, aus dem das Trägersubstrat besteht, die wenigstens eine Öffnung wenigstens teilweise durchdringt. Hierdurch ist eine verbesserte mechanische Verankerung der jeweiligen Elektrodenstreifen mit dem Trägersubstrat geschaffen.

Eine weitere Möglichkeit für eine dauerstabile Verbindung zwischen den Elektrodenflächen bzw. Elektrodenstreifen und dem biokompatiblen Polyimid bzw. Polymermaterial des Trägersubstrats spiegelt sich in einer speziellen Ausbildungsform der Elektrodenflächen bzw. Elektrodenstreifen wieder, sowie einer dadurch möglichen speziellen Integration der Elektroden in das Trägersubstrat. Hierzu weisen insbesondere die ersten und dritten Elektrodenstreifen jeweils eine metallische Basisplatte mit einer ebenen Ober- und Unterseite auf, mit wenigstens einem, vorzugsweise eine Vielzahl die Oberseite der Basisplatte orthogonal lokal überragenden Strukturelementen, die vorzugsweise säulen-, rippen-, hülsen oder stegartig ausgebildet sind. Die metallische Basisplatte ist vollständig von dem biokompatiblen Polymer des Trägersubstrats umschlossen, mit Ausnahme eines ersten Oberflächenbereiches des wenigstens einen Strukturelementes, der der Trägersubstratoberfläche zugewandt orientiert ist und diese nicht überragt. Somit reduziert sich die frei an der Trägersubstratoberfläche zugängliche Elektrodenkontaktfläche, jedoch ist aufgrund der hermetischen Umschließung der Basisplatte sowie der einstückig daran verbundenen Strukturelemente mit Ausnahme der der Trägersubstratoberfläche zugewandt orientierten Oberflächenbereiche vollständig vom biokompatiblen Polymer des Trägersubstrates umschlossen. Ein Eindringen von milieubedingter Flüssigkeit bzw. Feuchtigkeit zwischen den Elektrodenstreifen und dem biokompatiblen Polymer des Trägersubstrates wird erheblich erschwert, so dass Degradationserscheinungen weitgehend ausgeschlossen werden können. In einer weiteren bevorzugten Ausführungsform ist vorzugsweise zwischen der Unterseite der metallischen Basisplatte und dem biokompatiblen Polymer des Trägersubstrates eine Haftvermittlerschicht oder eine Haftvermittlerschichtanordnung eingebracht, die mögliche feuchtigkeitsbedingten Ablöseerscheinungen entgegen tritt.

Die lösungsgemäße implantierbare Anordnung ermöglicht in vorteilhafterweise die Durchführung eines Verfahrens zur ortsselektiven Erfassung neuronaler elektrischer Signale, die sich längs wenigstens einer in einem Nervenfaserbündel enthaltenden Nervenfaser eines menschlichen oder tierischen lebenden Organismus ausbreiten, sowie zur selektiven elektrischen Stimulation der wenigstens einen Nervenfaser. Insbesondere im Falle der elektrischen Stimulation von afferenten Nervenfasern, d.h. Nervenfasern, längs denen neuronale elektrische Signale zum Gehirn geleitet werden, ruft die lösungsgemäße, elektrische Stimulation keine oder keine signifikante Irritation der Gehirnfunktion hervor, zumal eine Unterscheidung von natürlichen, neuronalen elektrischen Signalen und elektrischen Stimulationssignalen im Gehirn nicht möglich ist. Das lösungsgemäße Verfahren zeichnet sich durch die folgenden Verfahrensschritte aus:
Zunächst gilt es sich längs einer Nervenfaser ausbreitende neuronale elektrische Signale, die es zu beeinflussen gilt, ortsselektiv zu erfassen. Dieser Schritt kann mit einer an sich bekannten Elektrodenanordnung vorgenommen werden. Auf der Grundlage der erfassten, natürlichen, neuronalen elektrischen Signale werden "künstliche" elektrische Signale generiert, deren Signaldauer und zeitlicher Amplitudenverlauf den erfassten, natürlichen neuronalen elektrischen Signalen entsprechen. Je nach einer therapeutischen Zielvorgabe, bspw. Blutdrucksenkung oder -erhöhung, werden die "künstlich" generierten elektrischen Signale derart modifiziert, in dem die Amplitude zumindest innerhalb eines zeitlichen Teilbereiches der Signaldauer der elektrischen Signale angehoben oder abgesenkt wird. Vorzugsweise wird der zeitliche Amplitudenverlauf des elektrischen Signals gesamtheitlich, d.h. über die gesamte Signaldauer des elektrischen Signals einheitlich angehoben oder abgesenkt. Durch diese Maßnahme werden elektrische Stimulationssignale erhalten, die an die Nervenfaser in zeitlicher Phase mit den neuronalen elektrischen Signalen appliziert werden. Dies bedeutet, dass die natürlichen, neuronalen elektrischen Signale zeitlich kohärent, d.h. entsprechend ihrer zeitlichen Signaldauer und zeitlichen Abfolge längs der Nervenfaser mit jeweils einem elektrischen Stimulationssignal überschrieben werden. Durch diesen Vorgang des zeitlich kohärenten Überschreibens wird die dem natürlichen, neuronalen elektrischen Signal innewohnende Information durch eine künstlich erzeugte, dem elektrischen Stimulationssignal eingeprägte Information ersetzt.

Da sich die elektrischen Stimulationssignale längs der afferenten Nervenfaser in gleicher zeitlicher Abfolge und mit gleicher zeitlicher Signaldauer wie die ursprünglichen neuronalen Signale ausbreiten, ist eine Unterscheidung von neuronalen elektrischen Signalen und elektrischen Stimulationssignalen im Gehirn nicht möglich ist.

Um zu vermeiden, dass sich die an die jeweilige Nervenfaser applizierten elektrischen Stimulationssignale bidirektional längs der Nervenfaser ausbreiten, sieht eine bevorzugte Ergänzung vor, dass zeitlich vor und/oder während des Applizierens jeweils eines elektrischen Stimulationssignals an die Nervenfaser ein elektrisches Inhibitionssignal an die Nervenfaser derart appliziert wird, so dass sich das elektrische Stimulationssignal nur unidirektional längs der Nervenfaser ausbreiten kann. Die Unterdrückung der Signalausbreitung in die unerwünschte Nervenfaserrichtung erfolgt vorzugsweise mit Hilfe einer zusätzlichen, zur elektrischen Applikation der elektrischen Stimulationssignale dienenden Elektrodenanordnung, separaten Elektrodenanordnung, die benachbart zum Ort der Applikation der elektrischen Stimulationssignale längs der Nervenfaser und abgewandt zur Ausbreitungsrichtung der elektrischen Stimulationssignale angebracht wird.

Alle weiteren, die implantierbare Anordnung in vorteilhafter Weise ausbildenden Merkmale werden im Weiteren unter Bezugnahme auf die Figuren erläutert.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1: Blockdiagramm zur Illustration sämtlicher Komponenten der lösungsgemäßen implantierbaren Anordnung,
- Fig. 2a,b: implantierbare Elektrodenanordnung gemäß Stand der Technik,
- Fig. 3.: Zeitdiagramme zur Illustration des EKG-Signals und dem mit dem Blutdruck korrelierten neuronalen Zeitsignal,
- Fig. 4: Darstellung zur Erläuterung des sich aus n Einzelpulsen zusammensetzenden Stimulationssignals sowie zur Erläuterung eines Einzelpulses,
- Fig. 5: Darstellung zweier alternativer Betriebsweisen der implantierbaren Anordnung zur Blutdruckregelung,
- Fig. 6: Draufsicht einer schematisierten implantierbaren Elektrodenanordnung mit einer dritten Elektrodenanordnung zu Inhibition selektiver Nervenfasern,
- Fig. 7a: Darstellung eines Elektrodenstreifens mit Öffnung,
- Fig. 7b: detaillierte Darstellung eines in das Trägersubstrat integrierten Elektrodenstreifens,
- Fig. 7c: Alternative Ausbildung eines Strukturelementes,
- Fig. 8a-f: Darstellungen einer die implantierbare Elektrodenanordnung zusätzlich verstärkende Manschette,
- Fig. 9: Hydraulische Applikationsstruktur der implantierbare Elektrodenanordnung, und
- Fig. 10: Flußdiagramm zur Durchführung der elektrischen Stimulation einer Nervenfaser.

### Wege zur Ausführung der Erfindung, gewerbliche Verwendbarkeit

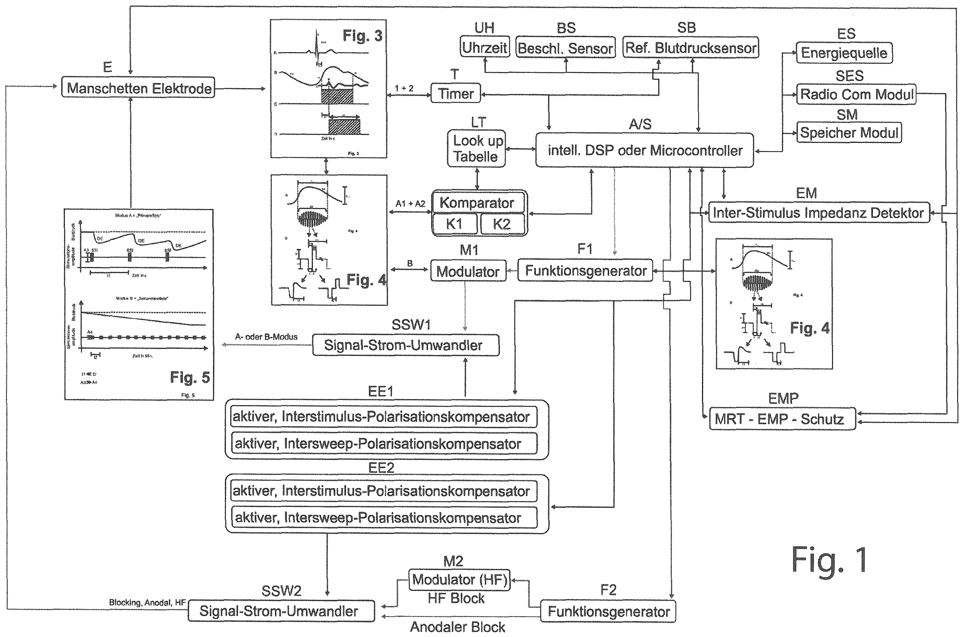

Figur 1 stellt ein Blockschaltbild dar, das die einzelnen Komponenten der implantierbaren Anordnung zeigt, die jeweils über zumeist bidirektionale elektrische Kommunikationspfade, siehe Verbindungspfeildarstellungen, miteinander verbunden sind. Die einzelnen Kommunikationspfade können in Form drahtgestützter oder drahtloser Verbindungsleitungen realisiert sein, über die elektrische Signale zur informellen Datenübertragung sowie auch elektrischen Energieübertragung bidirektional übertragbar sind.

Die Hauptkomponenten der implantierbaren Anordnung betreffen die implantierbare Elektrodenanordnung E, die Auswerte-/Steuereinheit A/S, eine mit der Auswerte-/Steuereinheit A/S elektrisch verbundene, erste Komparatoreinheit K1, ein ebenso mit der Auswerte-/Steuereinheit A/S verbundener erster Funktionsgenerator F1 sowie ein sowohl mit dem ersten Funktionsgenerator F1 mittel- oder unmittelbar sowie mit der implantierbaren Elektrodenanordnung E elektrisch verbundener, erster Signal-Stromwandler SSW1.

In einer ersten Ausführungsform entspricht die implantierbare Elektrodenanordnung E einer an sich bekannten Elektrodenanordnung, wie sie in Figur 2a, b illustriert ist, die manschettenartig um ein Nervenfaserbündel applizierbar ist, wobei zu Zwecken der selektiven Detektion elektrisch neuronaler Zeitsignale vorzugsweise als Tripolanordnung ausgebildete erste Elektrodenstrukturen 3 vorgesehen sind, die jeweils axial beidseitig von im implantierten Zustand ringförmigen ersten Elektrodenstreifen 5 begrenzt sind, siehe hierzu Fig. 2a, b.

Zusätzlich sieht die implantierbare Elektrodenanordnung E wenigstens eine Elektrode zur Erfassung des EKG-Signals vor, beispielsweise ist ein derartiger EKG-Signalabgriff mit Hilfe der Referenzelektroden 12 gemäß der in Figur 2a illustrierten, implantierbaren Elektrodenanordnung möglich.

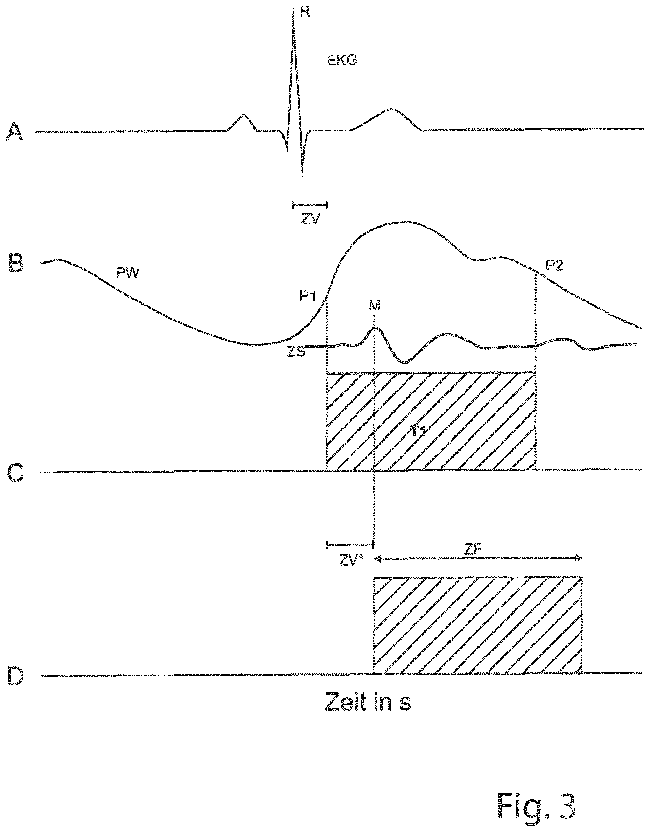

Die mit Hilfe der Elektrodenanordnung E detektierten elektrischen Informationen, sowohl das EKG-Signal als auch das mit dem Blutdruck korrelierte neuronale elektrische Zeitsignal betreffend, werden der Auswerte-/Steuereinheit A/S zeitaufgelöst zur weiteren Auswertung zugeführt. Vorzugsweise dient eine Timereinheit T zur zeitaufgelösten Erfassung und Übertragung der elektrischen Signale an die Auswerte-/Steuereinheit A/S. Für das weitere bessere Verständnis der Funktionsweise der implantierbaren Anordnung seien in Zusammenhang mit Figur 3 die zeitlichen Zusammenhänge zwischen einem mit Hilfe der im Bereich der Halsschlagader um den Nervus Vagus applizierten zweiten Elektrodenanordnung erfassten EKG-Signal (siehe A), der natürlichen Pulswelle bzw. Blutdruckwelle PW durch die Aorta (siehe B), die bspw. mit einem dedizierten Blutdrucksensor innerhalb der Aorta in unmittelbarer Herznähe erfassbar ist, sowie einem aus den neuronalen, elektrischen Signalen abgeleiteten, mit dem Blutdruck korrelierten neuronalen Zeitsignal ZS (siehe C), dargestellt.

Durch die mechanische Pulswelle PW werden die Barorezeptoren in der Aortenwand gereizt, wodurch die Barorezeptoren frequenzkodiert in Abhängigkeit der Stärke der Pulswelle PW neuronale, elektrische Signale aussenden. Diese synchrone Reizung vieler hunderte von Barorezeptoren erzeugt in Summe das neuronale, elektrische Signal, welches über die um den Nervus Vagus applizierte Manschettenelektrode abgegriffen werden kann.

Für eine technische Stimulierung des Nervus Vagus zu Zwecken einer therapeutisch motivierten Signalüberschreibung der natürlichen neuronalen elektrischen Signale, die längs des Nervus Vagus zum Gehirn gerichtet sind, bedarf es jedoch der Berücksichtigung wenigstens zweier Zeitverzögerungseffekte, die es bei der technischen Stimulation zu berücksichtigen bzw. zu kompensieren gilt, zumal die Stimulation so natürlich wie nur möglich vorgenommen werden soll, um zu vermeiden, dass das Gehirn durch den technischen Signaleingriff nachhaltig irritiert wird:
Dies betrifft zum einen den zeitlichen Versatz ZV zwischen dem Beginn des EKG-Signals bzw. der R-Zacke des EKG-Signals, siehe Fig. 3A, und dem Anstieg der Blutdruckwelle PW in der Aorta, siehe Punkt P1 in Fig. 3B. Zum anderen betrifft dies den zeitlichen Versatz ZV* zwischen der Reizung und Transduktion der Barorezeptoren und dem neuronalen, elektrischen Zeitsignal ZS, bis es den Bereich erreicht hat, an dem die Manschettenelektrode E das natürliche Blutdrucksignal überschreiben soll. Dieser Zeitversatz ZV* wird typischerweise zwischen dem Punkt P1 und einem ersten Maximum M des neuronalen elektrischen Zeitsignales ZS gewählt.

Die neuronalen, elektrischen Zeitsignale ZS können unterschiedlich in Erscheinung treten, in aller Regel weisen sie eine sog. "Mexican Hat"-Form auf und bestehen somit aus mehreren "Schwingungen". Dies erscheint zunächst verwunderlich, zumal das Pulswellensignal PW von den Barorezeptoren nicht in "Wellenform" kodiert wird. Der Grund hierfür liegt in der tripolaren Ausgestaltung der ersten Elektrodenanordnung. So läuft das "eingipfelige", natürliche, neuronale elektrische Pulswellensignal längs des Nervus Vagus in Längsrichtung an den drei Elektrodenstrukturen der ersten Elektrodenanordnung vorbei und vermag diese zeitlich nacheinander zu polarisieren. Auf diese Weise wird das monophasische neuronale elektrische Signal in das mehrphasige, neuronale elektrische Zeitsignal ZS umgesetzt.

Das abgeleitete, mehrphasige, neuronale, elektrische Zeitsignal ZS liegt zudem zeitlich stets zwischen einem charakteristischen Flankenanstiegspunkt P1 und einem Flankenabstiegspunkt P2 der Pulswelle PW, d.h. innerhalb des Zeitfensters T1, das der zeitlichen Dauer der Pulswelle PW entspricht.

Unter Berücksichtigung der vorstehend erläuterten Zeitversätze ZV und ZV* gilt es innerhalb des Zeitfensters ZF, siehe Fig. 3D, das elektrische Stimulationssignal zu generieren und über die erste Elektrodenanordnung an den Nervus Vagus zu Zwecken einer selektiven Stimulation der wenigstens einen barorezeptiven Nervenfaser zu applizieren.

Die Ermittlung des mit dem Blutdruck korrelierten neuronalen elektrischen Zeitsignals ZS (siehe Fig. 3 C) bedarf im Unterschied zur Erfassung und messtechnischen Nutzung des EKG-Signals einer speziellen Signalverarbeitung bzw. Signalaufbereitung, zumal sich der Signalpegel der messtechnisch erfassten, mit dem Blutdruck korrelierten Zeitsignale ZS nicht vom umgebenden elektrischen Rauschniveau abzuheben vermag. Hierbei werden die mit Hilfe der implantierbaren Elektrodenanordnung gewonnenen, neuronalen, elektrischen Signale vorzugsweise einer kohärenten Mittelung vorzugsweise mit dem EKG-Signal als Trigger unterzogen, wobei all jene Signalanteile einer zusätzlichen Verstärkung unterzogen werden, die reproduzierbar dem Blutdruck folgen. Weitere Einzelheiten hierzu können dem eingangs zitierten Beitrag von Dennis T. T. Plachta, Oscar Cota, Thomas Stieglitz, Mortimer Gierthmuehlen, "Selektive Ableitung und Stimulation für ein blutdrucksenkendes Implantat unter Verwendung von Vielkanal-Cuff-Elektroden", tm - Technisches Messen, 2013, Vol.80 (5), pp.163-172 entnommen werden.

Mit Hilfe des so gewonnenen, mit dem Blutdruck korrelierten, neuronalen, elektrischen Zeitsignals ZS, das in Figur 3C in seinem zeitlich variierenden Amplitudenverlauf dargestellt ist, ist der zeitliche Versatz ZV zum EKG-Signal (siehe Figur 3A) zu ermitteln. An dieser Stelle sei festgehalten, dass das mit Hilfe der implantierbaren Anordnung ermittelbare Blutdruck-korrelierte, neuronale, elektrische Zeitsignal ZS lediglich einem relativen Blutdruckwert entspricht, d.h., die elektrischen Potenziale steigen und fallen mit dem Blutdruck, wohingegen das Zeitsignalmaximum keinem absoluten Blutdruck in mmHg darstellt. Zur absoluten Blutdruckwertbestimmung ist es daher erforderlich, einen zusätzlichen externen oder internen Referenzsensor vorzusehen, mit dem der absolute Blutdruck erfassbar ist. Zu Zwecken der Kalibrierung des mit Hilfe der implantierbaren Elektrodenanordnung gewonnenen Zeitsignals ZS eignet sich vorzugsweise ein ebenfalls implantierbarer, technischer Blutdrucksensor, beispielsweise ein an sich bekannter Tipkatether, oder eine extrakorporal anlegbare Blutdruckmanschette. In Figur 1 ist hierzu der Referenzblutdrucksensor SB als weitere Komponente dargestellt, die nicht notwendigerweise als implantierbare Einheit ausgebildet sein muss. Vorzugsweise werden die mit Hilfe des Referenzblutdrucksensors SB ermittelten absoluten Blutdruckwerte ebenfalls zeitaufgelöst der Auswerte-/Steuereinheit A/S zugeleitet. Für die entsprechende Zeiterfassung dient eine mit dem Referenzblutdrucksensor SB in Datenaustausch stehende Uhr UH.

Um die barorezeptiven Nervenfasern innerhalb des von der implantierbaren Elektrodenanordnung E manschettenartig umfassten Nervenbündels in einer für eine effiziente Blutdrucktherapie erforderlichen Weise elektrisch zu stimulieren, bedarf es einer Analyse des EKG-Signals sowie des mit dem Blutdruck korrelierten neuronalen elektrischen Zeitsignals. Die diesbezügliche Auswertung erfolgt in der Auswerte-/Steuereinheit A/S mit dem Ziel, exakt jene Zeitpunkte zu ermitteln, an denen das Gehirn die über die barorezeptiven Nervenfasern übertragenen barorezeptiven Signale erwartet. Die technische elektrische Stimulation der die Blutdrucksignale übertragenden Nervenfasern soll zudem mit der natürlichen Blutdrucksignalzuführung in Bezug auf Zeitpunkt, Zeitdauer sowie auch der sich qualitativ zeitlich ändernden Signalform übereinstimmen. Als Timer- bzw. Triggersignal wird das EKG-Signal monopolar als Artefakt, beispielsweise über die Referenzelektroden 12 (siehe Figur 2a) erfasst und als Zeitsignal an die Auswerte-/Steuereinheit A/S übertragen. In der Auswerte-/Steuereinheit A/S wird im Weiteren der Zeitversatz ZV zwischen dem EKG-Signal und der natürlichen Pulswelle PW bestimmt. Hierzu dient insbesondere der Zeitunterschied zwischen der R-Zacke des EKG-Signals und einem charakteristischen Signalflankenpunkt P1 längs der aufsteigenden Anfangsflanke der Blutdruckwelle PW. Der Zeitversatz ZV wird bei einem Menschen mit normalem Puls (65 BPM) bei < 200 ms liegen (Fig. 3A, siehe Zeitachse in Sekunden). Ferner wird im Rahmen der Auswerte-/Steuereinheit A/S die charakteristische Pulsdauer T1 der Pulswelle PW gemessen, die durch den zeitlichen Abstand des ersten Signalflankenpunktes P1 sowie eines zweiten Signalflankenpunktes P2 längs der abfallenden Signalflanke gegeben ist.

Im Weiteren wird innerhalb der Auswerte-/Steuereinheit A/S unter Berücksichtigung biologisch bedingter Verzögerungen, beispielsweise bedingt durch die Transduktion eines mechanischen Ereignisses (Pulswelle) in ein bioelektrisches Signal und/oder durch die Umwandlung eines technischen Stromsignals in ein bioelektrisches, neuronales Potential und/oder durch die charakteristischen Leitungsgeschwindigkeiten längs neuronaler Fasern hervorgerufener Zeitverzögerungen, ein korrigierter Zeitversatz ZV* ermittelt, der bei der Generierung eines Stimulationssignals berücksichtigt wird.

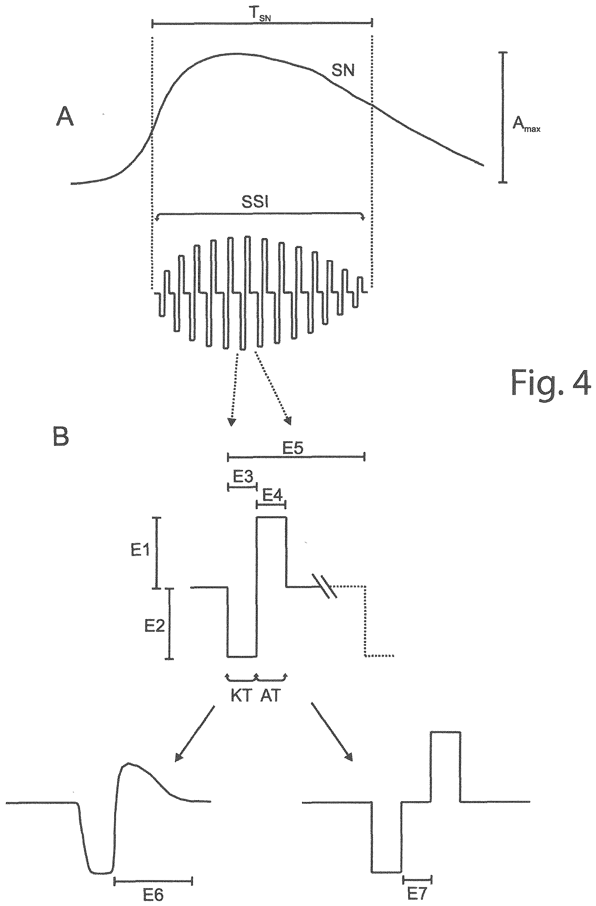

So gilt es, die wenigstens eine barorezeptive Nervenfaser innerhalb eines bestimmten Zeitfensters ZF, das in einem definierten Zeitversatz ZV + ZV* zum erfassten EKG-Signal liegt, elektrisch zu stimulieren. Dies erfolgt mit Hilfe des Funktionsgenerators F1, der ein sich aus einer Vielzahl n Einzelpulsen zusammensetzendes und in Phase und zeitlichem Amplitudenverlauf an das natürliche Blutdruck korrelierte, neuronale elektrische Signal SN (siehe Figur 4 A) angepasstes Stimulationssignal SSI generiert. In diesem Zusammenhang sei erwähnt, dass die sich längs barorezeptiver Nervenfasern ausbreitenden, natürlichen neuronalen, elektrischen Signale Sn in Bezug auf Zeitdauer T_{SN}, und Amplitudenform der Form und Pulsdauer einer Blutdruck- bzw. Pulswelle PW gleichen.

Der Funktionsgenerator F1 moduliert dabei die Amplituden der n Einzelpulse EP (in Figur 4A setzt sich das Stimulationssignal SSI aus 13 Einzelpulsen EP zusammen, in Wirklichkeit werden 100 bis 200 Einzelpulse ein Stimulationssignal ergeben) und bildet somit den mit Hilfe der implantierbaren Elektrodenanordnung E erfassten biologischen Druckverlauf im Stimulationssignal SSI - als umhüllende Funktion- in etwa ab. Zur Festlegung des zeitlich variablen Amplitudenverlaufes des Stimulationssignals SSI, d.h. der aufeinander abgestimmten Amplituden der Einzelpulse EP innerhalb eines Stimulationssignals - dienen die Pulsdauer T1 des mit dem Blutdruck korrelierten Zeitsignals ZS sowie dessen maximale Amplitude Aₘₐₓ. In vorteilhafter Weise werden die Pulsdauer T1 sowie die maximale Amplitude Aₘₐₓ innerhalb der ersten Komparatoreinheit K1 ermittelt.

Jeder Einzelpuls EP besitzt charakteristische Kennwerte, die aus Figur 4 B entnehmbar sind. So weist jeder Einzelimpuls EP einen kathodischen Signalteil KT und einen anodischen Signalteil AT auf. Der anodische Signalteil AT jedes Einzelimpulses EP verfügt über eine anodische Amplitude in Ampere E1 sowie über eine anodische Pulsbreite E4. Gleichfalls verfügt der kathodische Signalteil KT über eine kathodische Amplitude in Ampere E2 sowie eine kathodische Pulsbreite E3. Die Wiederholrate E5 wird in Hz bemessen. Nicht notwendigerweise muss die Wiederholrate E5 einer fixen Frequenz entsprechen, vielmehr hat sich gezeigt, dass neuronale Systeme, d.h. neuronale Nervenfasern, am besten stimuliert werden können, wenn äußere elektrische Aktivierungen dem natürlichen typischen Muster neuronaler Aktivität folgen bzw. entsprechen, d.h. einer Verteilungsfunktion, vorzugsweise einer Poisson-Verteilung unterliegen. Die Erzeugung der sich aus der Vielzahl n Einzelpulse EP zusammensetzenden Stimulationssignals SSI erfolgt mit dem ersten Funktionsgenerators F1 vorzugsweise derart, dass die beiden Phasen jedes Einzelpulses EP, also die Flächen des anodischen AT und des kathodischen Signalteils KT gleich sind, da sich ansonsten die Elektrodenkontakte, d.h. zumindest die Elektrodenflächen 4 der tripolaren Elektrodenstrukturen 3, siehe Fig. 2, polarisieren würden, wodurch nachfolgende elektrische Stimulationen mit einer signifikant geringeren Wirksamkeit an die wenigstens eine selektierte Nervenfaser innerhalb des Nervenfaserbündels appliziert werden können. Weiterhin kann Ladungsungleichgewicht zur Korrosion durch Redoxreaktionen führen, wenn sich durch die Polarisation ein Gleichspannungspotential aufbaut und dieses die Grenzen des Wasserfensters überschreitet. Die einzelnen anodischen und kathodischen Signalteile AT, KT jedes Einzelpulses EP werden durch den ersten Funktionsgenerator F1 in Form rechteckförmiger Teilsignale erzeugt.

Es hat sich zudem als vorteilhaft erwiesen, dass sich eine gewisse "Abrundung" der Signalflanken jedes Einzelimpulses EP in vorteilhafter Weise hinsichtlich einer Reduzierung von an den Metallkontakten der einzelnen Elektroden der implantierbaren Elektrodenanordnung auftretenden Korrosionseffekten auswirkt, wodurch ihre Lebensdauer verbessert werden kann. Eine derartige technische Signalflankenabrundung, insbesondere der Repolarisationsflanke E6, die in der schematischen Darstellung in Figur 4B unten links illustriert ist, wirkt sich neben den vorstehend erläuterten, verbesserten elektrochemischen Eigenschaften auch günstiger in Bezug auf die biologische Wirksamkeit bei der elektrischen Stimulation der wenigstens einen selektierten Nervenfaser aus. Hierzu ist dem ersten Funktionsgenerator F1 unmittelbar ein erster Modulator M1 nachgeschaltet, siehe Figur 1. Der Modulator M1 vermag vornehmlich den rechteckförmigen, repolarisierenden Signalteil AT zeitlich gegenüber dem polarisierenden Signalteil KT zu verlängern bzw. zu glätten, wobei beide Signalteile AT, KT über einheitliche, d.h. gleiche Signalstärken verfügen, um eine vollständige Repolarisierung der Elektrodenflächen zu ermöglichen. Alternativ oder in Kombination mit der vorstehenden Maßnahme der Abrundung zumindest des repolarisierenden Signalteils AT sieht eine weitere vorteilhafte Einflussnahme des ersten Modulators M1 auf jeden der einzelnen, vom ersten Funktionsgenerator F1 generierten Einzelpulse EP eine zeitliche Entkopplung zwischen dem kathodischen Signalteil KT und dem anodischen Signalteil AT durch eine zeitliche Pause E7 vor (siehe Figur 4, untere rechte Darstellung). Hierdurch wird eine extrem lange Signalflanke zwischen dem kathodischen und anodischen Signalteil KT, AT vermieden werden, wodurch unerwünschte neurostimulierende Artefakte ausgeschlossen werden können. Selbstverständlich ist es zudem möglich, die zeitliche Länge der Pause E7 zu modulieren.

Sämtliche der vorstehend beschriebenen und aus Figur 4 entnehmbaren Einzelpulscharakteristika E1 bis E7 können mit Hilfe des ersten Modulators M1 individuell aufeinander abgestimmt und eingestellt werden.

Die implantierbare Anordnung vermag in Abhängigkeit individueller Regelungserfordernisse zu Zwecken der Nivellierung des Blutdruckes autonom entscheiden, ob, wann und in welcher Stärke und Dauer elektrische Stimulationssignale SSI an die wenigstens eine barorezeptive Nervenfaser appliziert werden sollen. Hierzu sieht die implantierbare Anordnung gemäß Figur 1 wenigstens eine zweite Komparatoranordnung K2 vor, die mit der Auswerte-/Steuereinheit A/S elektrisch verbunden ist. Die zweite Komparatoranordnung K2, die mit der ersten Komparatoreinheit K1 baulich zusammengefasst ausgebildet sein kann, vergleicht wenigstens einen charakteristischen Signalpegel des mit dem Blutdruck korrelierten neuronalen Zeitsignals ZS mit einem Referenzsignal, das vorzugsweise in einer Lockup-Tabelle LT abgelegt ist, die sowohl mit der Auswerte-/Steuereinheit A/S sowie auch mit der Komparatoreinheit K2 verbunden ist, und generiert einen charakteristischen Differenzpegel, auf dessen Grundlage die Auswerte-/Steuereinheit A/S wenigstens den zeitlichen Amplitudenverlauf, d.h. den zeitlich variablen Amplitudenpegel des Stimulationssignals SSI festlegt. Letztlich können in vorteilhafter Weise auf der Grundlage wenigstens des generierten Differenzpegelwertes sämtliche Stimulationsparameter E1 bis E7 aufeinander variabel abgestimmt werden, so dass sämtliche Einzelpulsformen individuell gewählt werden können, um ein an die wenigstens eine barorezeptive Nervenfaser zu applizierendes Stimulationssignal SSI zusammenzusetzen.

Selbstverständlich können in vorteilhafter Weise neben dem in der Lockup-Tabelle LT abgespeicherten Referenzsignal weitere, den physiologischen Zustand des jeweiligen Patienten charakterisierende Informationen zu Zwecken der elektrischen Stimulation der wenigstens einen barorezeptiven Faser abgespeichert werden, wie beispielsweise Informationen, die den Bewegungszustand des Patienten charakterisieren, der Differenzpegelwert, erfasster absoluter Blutdruck etc..

So verfügt die implantierbare Anordnung in einer vorteilhaften Ausführungsform über einen Beschleunigungssensor BS, der vorzugsweise in dem implantierbaren Modul integriert ist, in dem die Auswerte-/Steuereinheit A/S die erste und zweite Komparatoreinheit K1, K2, der erste Funktionsgenerator F1 sowie der erste Signal-Stromwandler SSW1 zusammengefasst sind. Der Beschleunigungssensor BS ist elektrisch mit der Auswerte-/Steuereinheit A/S verbunden und vermag somit die generierten Beschleunigungsinformationen zur weiteren Auswertung der Auswerte-/Steuereinheit A/S zuzuleiten. Ebenso ist es möglich, einen extrakorporal am Patienten angebrachten Beschleunigungssensor zu nutzen, dessen Beschleunigungsinformationen drahtlos, beispielsweise per induktiver Datenkopplung, der Auswerte-/Steuereinheit A/S zuführbar sind. Der wenigstens eine, vorzugsweise triaxiale Beschleunigungs- bzw. Bewegungssensor BS vermag somit die körperliche Aktivität des jeweiligen Patienten zu erfassen, so dass bewegungsbedingte Blutdruckanstiege berücksichtigt werden können, die von Seiten der implantierbaren Anordnung als solche erkannt werden und nicht zu einer entsprechenden Blutdruck reduzierenden Stimulation der wenigstens einen barorezeptiven Nervenfaser führen.

Neben einer möglichen extrakorporalen Ausbildung und Anbringung eines triaxialen Beschleunigungs- bzw. Bewegungssensors können weitere extrakorporale Einheiten in vorteilhafter Weise vorgesehen sein, wie beispielsweise eine Energiequelle ES, ein Speichermodul SM sowie eine Signal- und Energieversorgungseinheit SES. Zur Übertragung sämtlicher elektrischer Signale sowie auch zur Übertragung von elektrischer Energie dient die drahtlose induktionsbasierte Signal- und Energieübertragungstechnik.

Sämtliche Informationen, die der Auswerte-/Steuereinheit A/S zugeleitet werden, so insbesondere die intrakorporal erfassten Blutdruck korrelierten neuronalen Zeitsignale ZS sowie auch sämtliche extrakorporal zugeführte Informationen können in der Lockup-Tabelle LT abgespeichert und entsprechend aktualisiert werden, so dass der der implantierbaren Anordnung zugrunde liegende Regelmechanismus stets auf aktualisierte Informationen zurückgreifen kann. Beispielsweise können auf diese Weise die seitens der mit Hilfe der Elektrodenanordnung E abgegriffenen Blutdruck korrelierten neuronalen Zeitsignale ZS, die lediglich relative Blutdrucksignale repräsentieren, mit aktuellen, absoluten Blutdruckwerten kalibriert werden, die mit Hilfe intrakorporaler oder extrakorporaler Blutdruckmesssysteme SB erfassbar sind. Ferner ermöglicht die lösungsgemäß ausgebildete, implantierbare Anordnung eine selbstregulative Überwachung der an der wenigstens eine barorezeptive Nervenfaser applizierten Stimulationssignale, indem mit Hilfe der Elektrodenanordnung E die organische Rückmeldung der vorgenommenen Stimulationen erfassbar ist, so dass eine so genannte "Closed-Loop-Regelfunktion" realisierbar ist. Alternativ oder in Kombination zu bzw. mit der vorstehend erwähnten Lookup Tabelle kann auch ein weiterer Speicherbereich für das Vorhalten von Informationen oder Signalen vorgesehen sein, so dass auch Signale als solche hinterlegt werden können, wenn beispielsweise ein Zustandsschätzer / Kalman-Filter zur Regelung benutzt wird und zeitlich zurückliegende Signale die Stellgröße für die Regelung mit beeinflussen sollen.

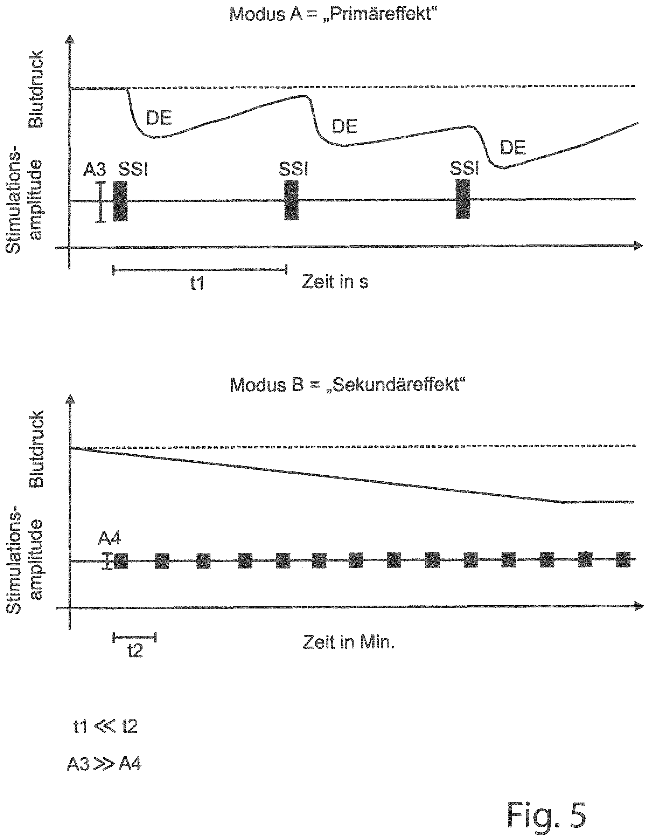

Unter Bezugnahme auf Figur 5 seien im Weiteren zwei unterschiedliche Blutdruckregelungsmodi erläutert, mit der die implantierbaren Anordnung eine Blutdruckbeeinflussung vornehmen kann. In beiden in Figur 5 dargestellten Diagrammen zeigt der jeweils obere Graph den Blutdruck längs zur Zeitachse t. Der jeweils untere Graph in beiden Diagrammen stellt schematisiert die Stimulationsamplitude jeweils eines Stimulationssignals SSI dar. Im Falle der in Figur 5 oberen dargestellten Blutdruckregelung ist zu erkennen, dass unmittelbar nach Aktivierung und Applizierung eines Stimulationssignals SSI, das eine Stimulationsamplitude A3 besitzt, eine den Blutdruck signifikant reduzierende Delle DE hervorgerufen wird. Wird das Stimulationssignal SSI mit einer zeitlichen Wiederkehr t1 wiederholt, so führt dies zu einer schnellen Blutdrucksenkung, bis ein erwünschter Blutdruckwert erreicht ist.

Der in der unteren Diagrammdarstellung illustrierte Blutdruckregelungsmodus B führt hingegen zu einer anderen Rückantwort der natürlichen, physiologischen Blutdruckregelung. So werden in diesem Fall die Stimulationssignale SSI mit einer sehr viel geringeren Stimulationsamplitude A4 aktiviert und appliziert, als im Falle der Stimulationsamplitude A3 im vorstehend erläuterten Regelungsmodus A. Durch eine derart geringe Stimulationssignalamplitude A4 wird keine akute Delle DE im Blutdruckwert erzielt. Werden die zeitlichen Abstände zwischen den einzelnen Stimulationssignalen SSI im Falle des Regelmodus B zudem geeignet groß gewählt (siehe Zeitachse in Minuten), d.h. sehr viel größer als im Falle des Modus A, so führt dies zu einer sehr langsamen aber stetigen Blutdruckabsenkung, wie dies aus der Blutdruckfunktion im Falle des Regelmodus B entnommen werden kann. Durch diesen, auch als "Sekundäreffekt" bezeichneten Regelmodus B, im Vergleich zu dem als "Primäreffekt" bezeichneten Regelmodus A, kann viel Energie zum Betrieb der implantierbaren Anordnung eingespart werden. Ferner ist die Belastung für das Nervengewebe sowie auch für die Elektroden erheblich geringer, zudem kann der Blutdruck behutsam eingeregelt werden. Sowohl die Stimulationsamplitude A4 als auch die zeitliche Wiederholrate t2 können individuell zum Einstellen eines gewünschten reduzierten Blutdruckes gewählt werden. Der als Regelmodus B bezeichnete modus operandi eignet sich bevorzugt zur Therapierung chronischer Hypertonie, wohingegen der als Primäreffekt bezeichnete Regelmodus A in Fällen einer hypertonen Krise zur Anwendung kommen sollte.

Die lösungsgemäße implantierbare Anordnung vermag autonom je nach Auftreten bestimmter Blutdrucksituationen zwischen beiden Regelmodi im Betrieb zu wechseln, d.h. gilt es möglichst schnell Blutdruckspitzen zu reduzieren, so eignet sich der Regelmodus A, gilt es hingegen langsame Blutdruckkorrekturen vorzunehmen, so wird Regelmechanismus B angewendet. Zur Entscheidung, welche der beiden Regelungsmechanismen zur Anwendung kommen, können sämtliche in der Lock-up-Tabelle aktuell erfassten sowie der Auswerte-/Steuereinheit A/S zugeführten Informationen verwertet werden.

Wie bereits in Verbindung mit Figur 4 erläutert, setzen sich die n Einzelpulse EP aus einem kathodischen und aus einem anodischen Signalteil KT und AT zusammen, so dass sich beide Polarisationssignalflächen gegenseitig vollständig aufheben (ladungsausgeglichene Stimulation, charge balanced stimulation), wodurch eine Restpolarisation der jeweils zur Stimulation beitragenden Elektroden vermieden werden soll. Trotz dieser Maßnahmen ist es möglich, dass eine, wenn auch nur geringe, Restpolarisation an den Elektroden nicht immer ausgeschlossen werden kann, wodurch folgende Stimulationseinzelpulse sowohl bezüglich ihrer Pulsform als auch Pulsstärke beeinträchtigt sein können. Diese unerwünschten, durch Restpolarisationen verursachten Effekte gilt es auszuschließen.

Hierzu sieht eine weitere bevorzugte Ausführungsform der lösungsgemäß ausgebildeten implantierbaren Anordnung eine Elektrodenimpedanzmesseinheit EM vor, siehe Figur 1, die Teil des implantierbaren Moduls ist oder an dem Trägersubstrat 1 angeordnet ist und mit der Elektrodenanordnung E sowie mit der Auswerte-/Steuereinheit A/S elektrisch verbunden ist. Die Elektrodenimpedanzmesseinheit EM ist derart ausgebildet, dass sie zwischen jedem der n Einzelpulse EP eine Impedanzmessung zumindest an den Elektroden der ersten Elektrodenanordnung vornimmt und damit ihre Polarisation misst. Die Elektrodenimpedanzmesseinheit EM ist ferner mit einer ersten Entpolarisierungseinheit EE1 verbunden, die ebenfalls Teil des implantierbaren Moduls ist oder an dem Trägersubstrat 1 angebracht ist. Die erste Entpolarisierungseinheit EE1 vermag im Falle einer durch die Elektrodenimpedanzmesseinheit EM festgestellten Restpolarisation an einzelnen Elektroden diese zu entpolarisieren, indem sie die entsprechenden Elektroden selektiv mittels elektrischer Signalbeaufschlagung kurzzeitig aktiviert. Die erste Entpolarisierungseinrichtung EE1 vermag darüber hinaus nicht nur mögliche Restpolarisationen an den bei der Stimulation beteiligten Elektroden zwischen jedem Einzelpuls zu erfassen und entsprechend zu beseitigen, vielmehr erfolgt eine entsprechende Polarisationsmessung und eine entsprechende aktive Entpolarisierung auch nach jedem einzelnen Stimulationssignal SSI. Im Ergebnis können sämtliche Einzelpulse EP frei oder zumindest weitgehend frei von Polarisationseffekten erzeugt werden, so dass jedes einzelne Stimulationssignal SSI unter gleichen elektrischen Verhältnissen mit Hilfe der Elektrodenanordnung generiert wird. Auf diese Weise kann ausgeschlossen werden, dass die Stimulationssignale in zeitlicher Abfolge ein unterliegendes Gleichspannungspotenzial aufbauen.

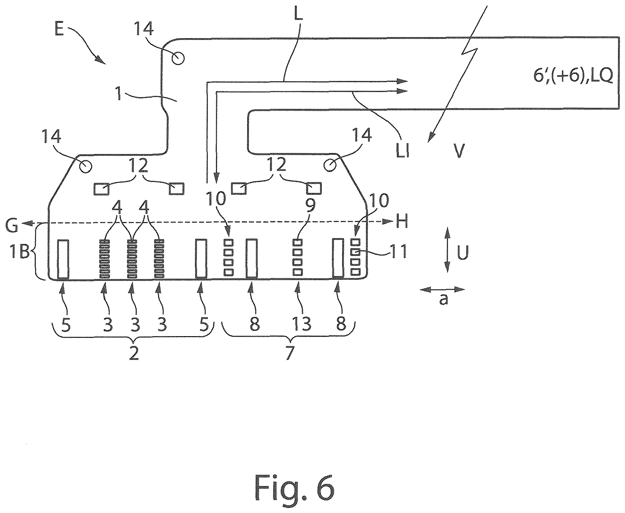

Die lösungsgemäß ausgebildete implantierbare Anordnung sieht in einer bevorzugten Ausführungsform eine gegenüber der in Figur 2a, b illustrierten implantierbaren Elektrodenanordnung E eine modifizierte Elektrodenanordnung E vor, die in Figur 6 illustriert ist, auf die weiter unten eingegangen wird, und die eine unerwünschte, aber mit der vorstehend beschriebenen Elektrodenanordnung mögliche Signalausbreitung längs der barorezeptiven Nervenfaser in Richtung Herz aktiv zu unterdrücken vermag.

Hierzu sieht die implantierbare Elektrodenanordnung an der im implantierten Zustand, dem Nervenfaserbündel zugewandt orientierten, geradzylinderförmigen Trägersubstratoberfläche eine dritte Elektrodenanordnung 7 zur Inhibition von sich unidirektional längs des Nervenfaserbündels ausbreitenden neuronalen elektrischen Signalen vor. Die dritte Elektrodenanordnung 7, auf die im Weiteren ausführlich Bezug genommen wird, wird ausschließlich bzw. vornehmlich in Verbindung mit der elektrischen Stimulation der wenigstens einen barorezeptiven Nervenfaser aktiviert. Hierzu ist ein zweiter Funktionsgenerator F2 vorgesehen, der ebenfalls im implantierbaren Modul integriert ist und zeitlich vor und/oder während des ermittelten Zeitfensters T1, siehe Figur 4, ein elektrisches, so genanntes Blockierungs- oder Inhibitionssignal generiert. Der zweite Funktionsgenerator F2, siehe Figur 1, ist ferner mittel- oder unmittelbar mit einem zweiten Signal-Strom-Wandler SSW2 verbunden, der das elektrische Inhibitionssignal an die dritten Elektrodenanordnung 7 zuführt. Gleichsam dem ersten Funktionsgenerator F1 ist auch der zweite Funktionsgenerator F2 in der Lage, Einzelpulse zu erzeugen, die jeweils aus einem polarisierenden und einem repolarisierenden, rechteckförmigen Teilsignal zusammengesetzt sind. Auch in diesem Fall neutralisieren sich beide Teilsignale hinsichtlich ihrer Polaritätsfläche, so dass nach jedem Einzelpuls möglichst keine Restpolarisation an der jeweiligen Elektrodenflächen 9 verbleibt.

Ferner ist zwischen dem zweiten Funktionsgenerator F2 und dem zweiten Signal-Strom-Wandler SSW2 ein zweiter Modulator M2 geschaltet, der einen dem rechteckpulsförmigen, repolarisierenden Teilsignal AT zugeordneten Signalflankenverlauf zeitlich gegenüber dem polarisierenden Teilsignal KT vergrößert und glättet und die beiden Teilsignalen zugeordnete Signalstärke vereinheitlicht. Die mit dem zweiten Modulator M2 verbundene Maßnahme wird aus den gleichen Gründen praktiziert, wie vorstehend erläutert zum ersten Modulator M1. Neben dem zweiten Funktionsgenerator F2 ist auch der zweite Modulator M2 in dem implantierbaren Modul integriert. Auch im Falle des zweiten Modulators M2 ist es optional zusätzlich möglich, bei jedem Einzelpuls das polarisierende Teilsignal zeitlich durch einen im zweiten Modulator M2 erzeugbaren Nullsignalpegel vom repolarisierenden Teilsignal zu trennen. Auf diese Weise wird eine lange steil abfallende Signalflanke zwischen beiden Teilsignalen vermieden, die möglicherweise zu irritierenden Inhibitionseffekten oder zusätzlicher Stimulation der Nervenfasern unter den außenliegenden Elektroden (sogenannte Anodenöffnungszuckung - anodic break excitation) führen könnte.

Mit Hilfe der dritten Elektrodenanordnung 7 kann sich die Inhibition entweder im Wege eines sog. anodalen Blockes oder durch Applikation von sinusförmigen Signalen von Frequenzen im Kilohertzbereich, dem sogenannten HF-Block, realisieren. Im Falle des anodalen Blocks wird wenigstens eine der dritten Elektrodenflächen anodisch polarisiert, wodurch ein am Ort der efferenten Nervenfaser vorherrschendes Spannungspotential erzeugt wird, durch das eine aktivierende Stimulation der entsprechenden Nervenfaser unterdrückt wird. In diesem Fall ist eine zusätzliche Modulation mittels des zweiten Modulators M2 nicht erforderlich. Ebenso kann eine Inhibition im Wege einer Hochfrequenzsignalapplikation erzielt werden, bei der an wenigstens einer ausgewählten dritten Elektrodenfläche ein hochfrequentes elektrisches Inhibitionssignal angelegt wird, wodurch die elektrischen Signalübertragungsmechanismen längs der efferenten Nervenfasern kurzzeitig zum Erliegen kommen.

Die dritte Elektrodenanordnung 7 zur gezielten Inhibition von sich unidirektional, vorzugsweise in Richtung des Herzens, längs der wenigstens einen selektiven barorezeptiven Nervenfaser ausbreitenden Stimulationssignale ist gleichfalls mit der Elektrodenimpedanzmesseinheit EM verbunden, um mögliche Restpolarisationen an den der dritten Elektrodenanordnung 7 zugehörigen Elektrodenflächen 9, 8 zu erfassen. Für eine entsprechende Entpolarisierung möglicher Restpolarisationen sorgt ebenso eine zweite Entpolarisierungseinrichtung EE2, die sowohl Restpolarisierungen zwischen Einzelpulsen als auch zwischen möglichen aufeinanderfolgenden Inhibitionssignalen vermittels dosierter elektrischer Aktivierung einzelner Elektroden zu beseitigen vermag.

Zum Zwecke des elektrischen Schutzes der implantierbaren Anordnung hinsichtlich EMP-Schutz sowie magnetischer Einkopplung durch MRT ist eine entsprechende Einheit EMP innerhalb des implantierbaren Moduls integriert. Diese Einheit überwacht die Eingänge der Elektrode und erlaubt eine Entkopplung im Falle von extern evozierten Potentialschwankungen. Zusätzlich verfügt die EMP Einheit über einen Magnetfeldsensor, welcher bei der Erfassung eines starken DC Feldes ein temporäres Selbstschutzprogramm aktiviert.

Figur 6 zeigt eine schematische Draufsicht einer bevorzugt ausgebildeten, implantierbaren Manschettenelektrode E, auf deren vorzugsweise aus Polyimid bestehenden Trägersubstrat 1, neben der zur ortsselektiven Detektion neuronaler elektrischer Signale sowie zur selektiven elektrischen Stimulation einzelner Nervenfasern vorgesehenen ersten Elektrodenanordnung 2 eine zur Inhibition wenigstens einer selektierten Nervenfaser dienende dritten Elektrodenanordnung 7 aufgebracht ist. Zur Vermeidung von Wiederholungen wird auf die Erläuterung der einzelnen Elektroden der ersten Elektrodenanordnung 2 sowie der zweiten Elektrodenanordnung 12 auf die vorstehende Beschreibung der Figuren 2a und b verwiesen.

Die zur Signalausbreitung längs efferenter, hier zum Herzen H, führende Nervenfasern inhibierende dritte Elektrodenanordnung 7 umfasst zwei axial beabstandete dritte Elektrodenstreifen 8, zwischen denen mittig eine dritte Elektrodenstruktur 13 vorgesehen ist, die aus vier separat zueinander angeordneten dritten Elektrodenflächen 9 besteht. Sämtliche Elektroden 8, 13 der dritten Elektrodenanordnung 2 sind über auf dem Trägersubstrat 1 aufgebrachte oder in dieses intergierte elektrische Leiterbahnen L mit der Auswerte-/Steuereinheit A/S verbunden bzw. verbindbar. Die elektrischen Leiterbahnen L können optional eine trennbare Verbindungsstruktur V aufweisen.

Optional umfasst die dritte Elektrodenanordnung 2 optische Lichtwellenleiteranordnungen 10, die jeweils vier in Umfangsrichtung U verteilt angeordnete, separate Lichtwellenleiteröffnungen 11 umfassen. Die Lichtwellenleiter LI zu den einzelnen Lichtwellenleiteröffnungen bzw. -aperturen 11 verlaufen innerhalb des Trägersubstrats 1 und können proximalseitig mit einer einheitlichen Lichtquelle LQ oder mit separaten Lichtquellen LQ unterschiedlicher Lichtwellenlängen kombiniert werden, um optogenetisch selektiv aktivierte Stimulierungen und/oder optisch aktivierte und selektive Inhibitionen längs bestimmter Nervenfasern hervorzurufen.

Die geometrische Form- und Größenwahl der einzelnen Elektroden, d. h. der ersten und dritten Elektrodenstreifen 5, 8 sowie der ersten und dritten Elektrodenflächen 4, 9 können grundsätzlich individuell aufeinander abgestimmt vorgenommen werden und richten sich insbesondere nach dem Durchmesser des Nervenfaserbündels, um das die implantierbare Manschettenelektrode E anlegbar ist. So entspricht die in Umfangsrichtung U orientierte Erstreckung der ersten und dritten Elektrodenstrukturen und Elektrodenstreifen sowie gegebenenfalls der optischen Lichtwellenleiteranordnungen 10 vorzugsweise der Länge des Umfangsrandes des mit der Manschettenelektrode E zu umwickelnden Nervenfaserbündels. Der axiale Abstand der tripolaren Elektrodenanordnung sollte vorzugweise auf den Durchmesser und den damit resultierenden Abstand der sogenannten Ranvierschen Schnürringe bei myelinisierten Nervenfasern der zu erregende Nervenfasern angepasst werden. In dem in Figur 6 dargestellten Ausführungsbeispiel sind die Elektroden als rechteckförmige Elektrodenflächen dargestellt. In vorteilhafter Weise, so insbesondere zu Zwecken der Vermeidung von an Elektroden-Rechteckkanten auftretenden Feldlinienverdichtungen, bietet es sich an die Elektrodenflächen zumindest mit abgerundeten Ecken auszubilden.

So gilt es beim Menschen bestimmte, große und myelinisierte Fasern zu inhibitieren oder zu aktivieren. Dies ist nur an den Stellen längs der Nervenfaser möglich, an denen diese Fasern nicht myelinisiert sind, d.h. an den sogenannten Ranvierschen Ringen. Mit zunehmendem Durchmesser der Nervenfaser sind die Intervalle, d.h. die axialen Abstände zwischen den Ranvierschen Ringen größer, dementsprechend gilt es den axialen Abstand zwischen beiden axial beabstandeten ersten Elektrodenstreifen 5 in etwa gleich lang wie der axiale Abstand der Ringe oder etwas größer zu wählen, um mit hinreichend großer statistischer Wahrscheinlichkeit auch die Ranvierschen Ringe sehr großer Fasern zu erreichen. Entsprechendes gilt vorzugsweise auch für die axiale Beabstandung der dritten Elektrodenstreifen 8.

Die axiale Gesamterstreckung der gesamten Manschettenelektrode E sollte an die intrakorporalen Größenverhältnisse des jeweiligen Nervenfaserbündels angepasst sein und typischer Weise 4 cm nicht überschreiten.

Die rückseitig an dem Trägersubstrat 1 angebrachten Referenzelektrodenflächen 12 dienen der Erfassung des EKG-Signals und bedarfsweise des intrakorporal erfassbaren Rauschpegels.

Zusätzlich verfügt das Trägersubstrat 1 über wenigstens einen, vorzugsweise zwei oder drei durch Metallringstrukturen verstärkte Öffnungen 14, die zur Befestigung der implantierten Elektrodenanordnung CF am Nervenfaserbündel dienen. Die Befestigung erfolgt mit Hilfe eines chirurgischen Fadens, der jeweils wenigstens einmal durch die Öffnungen 14 gefädelt und in dem das Nervenfaserbündel umgebenden Gewebe vernäht wird. Im Unterschied zu dem zu einem Geradzylinder aufgerollten Bereich 1B des Trägersubstrats, auf dem die ersten und zweiten Elektrodenanordnungen 2 und 7 aufgebracht sind, so dass sie das Epineurium des Nervenfaserbündels im implantierten Zustand oberflächig berühren, steht das sich an den Trägersubstratbereich 1B anschließende Trägersubstrat 1 in Art einer ebenen Fahne seitlich vom Nervenfaserbündel ab und ragt in das umliegende Gewebe hinein. Die Metallringstrukturen 14 sollen helfen die längs des chirurgischen Fadens wirkenden Befestigungskräfte mechanisch sicher aufzunehmen und einschneidende Schädigungen am Trägersubstrat zu verhindern.

Zur manschettenartigen Umwicklung der implantierbaren -Elektrodenanordnung E um ein nicht weiter dargestelltes Nervenfaserbündel ist die dritte Elektrodenanordnung 7 auf die zum Herzen führenden Seite H längs des Nervenfaserbündels anzuordnen. Die erste, der selektiven Detektion sowie auch selektiven Stimulation lokalisierter Nervenfasern dienende Elektrodenanordnung 2 ist längs des Nervenfaserbündels gehirnseitig G angebracht.

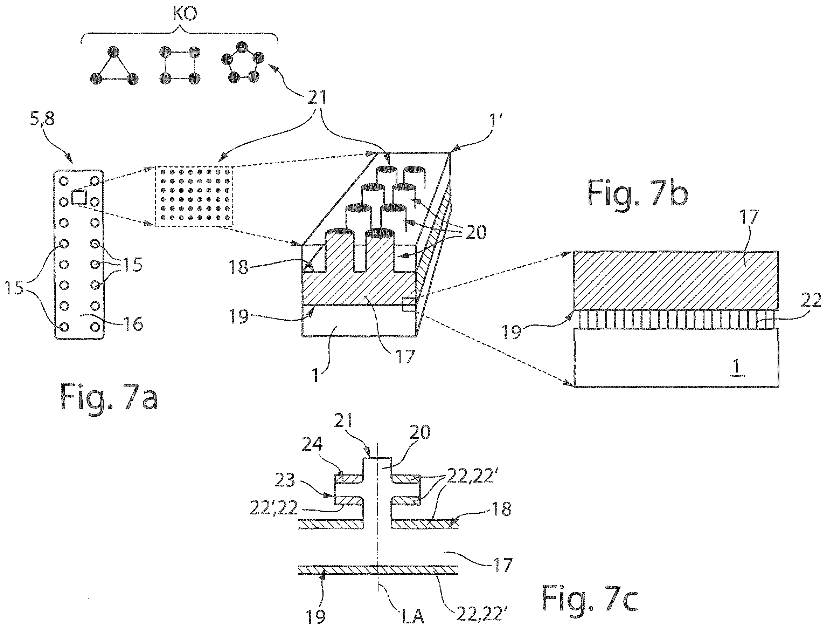

Vorzugsweise sind die ersten und dritten Elektrodenstreifen 5, 8 sowie die ersten und dritten Elektrodenflächen 4, 9 auf das Trägersubstrat aufgedampft oder aufgesputtert, eine galvanische Verstärkung ist denkbar. Auch Laserstrukturierung von dünnen Metallfolien ist als Technologie möglich. Für eine dauerhafte Fügung insbesondere der ersten und dritten Elektrodenstreifen 5, 8 an das Trägersubstrat 1 weisen die Elektrodenstreifen lokale Öffnungen 15 auf, siehe Figur 7a, durch die zumindest teilweise das Polymermaterial des Trägersubstrats 1 hindurchtritt bzw. hindurchragt. Die Elektrodenoberfläche 16 jeweils der ersten und dritten Elektrodenstreifen 5, 8 sind im Übrigen bündig zur Trägersubstratoberseite 1' angeordnet und kontaktieren die Oberfläche des Nervenfaserbündels unmittelbar.

Um die Fügung der Elektrodenstreifen 5, 8 dauerhaft zu verbessern, wird in einem bevorzugten Ausführungsbeispiel vorgeschlagen, die Elektrodenstreifen in der folgenden Weise weitgehend in das Trägersubstrat zu integrieren, siehe hierzu Figur 7b:
Die Elektrodenstreifen 5, 8 weisen jeweils eine metallische Basisplatte 17 auf, die eine Oberseite 18 und eine Unterseite 19 vorsieht. Einstückig mit der Oberseite 18 der Basisplatte 17 sind über die Oberseite 18, vorzugsweise über die gesamte Oberseite flächig verteilt, orthogonal erhabene Strukturelemente 20 vorgesehen, vorzugsweise in Form säulen-, rippen-, steg- oder hülsenartiger Fortsätze, die über einen der Trägersubstratoberfläche 1' zugewandten Oberflächenbereich 21 verfügen, der in unmittelbarer Anlage mit dem Epineurium des Nervenfaserbündels gelangen kann. Zusätzlich ist in vorteilhafter Weise eine Haftvermittlerschicht 22 zumindest zwischen der Unterseite 19 und dem die Basisplatte 17 umgebenden Polymermaterial des Trägersubstrats 1 vorgesehen. Die Haftvermittlerschicht 22 kann zudem auch auf der Oberseite 18 aufgebracht sein. Besonders geeignete Haftvermittlerschichten bestehen aus Siliziumcarbid (SiC) sowie Diamond like Carbon (DLC). Vorzugsweise werden die Elektrodenstreifen 5, 8 aus Iridiumoxid gefertigt, das zu jenen Materialien mit einer der höchsten Ladungsübertragungskapazitäten zu zählen ist.

Eine weitere verbesserte Variante zur Ausbildung der Strukturelemente 20, die verteilt auf der Oberseite der Basisplatte 17 aufgebracht sind, ist in Figur 7c illustriert. Figur 7c zeigt den Längsschnitt durch ein Strukturelement 20, das eine orthogonal zur Oberseite 18 der metallischen Basisplatte 17 orientierte Längserstreckung LA aufweist, längs der das Strukturelement 20 wenigstens einen zweiten Oberflächenbereich 23 vorsieht, der parallel zur Oberseite 18 der metallischen Basisplatte 17 orientiert ist und auf dem die Haftvermittlerschicht 22 oder eine Haftvermittlerschichtanordnung 22' aufgebracht ist. Der zweite Oberflächenbereich 23 ist vom ersten Oberflächenbereich 18 beabstandet angeordnet und getrennt durch die Haftvermittlerschicht (22) bzw. die Haftvermittlerschichtanordnung (22') vollständig von dem biokompatiblen Polymer umgeben. Der zweite Oberflächenbereich ist, wie aus der Figur 7c zu entnehmen ist, der Oberseite 18 der Basisplatte 17 zugewandt orientiert. Selbstverständlich ist es zusätzlich möglich und vorteilhaft die Haftvermittlerschicht 22 bzw. die Haftvermittlerschichtanordnung 22' sowohl an einem dritten Oberflächenbereich 24, der dem zweiten Oberflächenbereich 23 gegenüberliegt und/oder an der Ober- und/oder Unterseite 18, 19 der Basisplatte 17 vorzusehen.

Die Anzahl sowie auch Anordnung der einzelnen Strukturelemente 20 können beliebig gewählt werden, vorzugsweise eignen sich jedoch geometrisch geordnete Konstellationen KO, wie bspw. quadratische, pentagonale, hexagonale oder höherwertige Anordnungsmuster, wie dies aus der Figur 7b zu entnehmen ist.

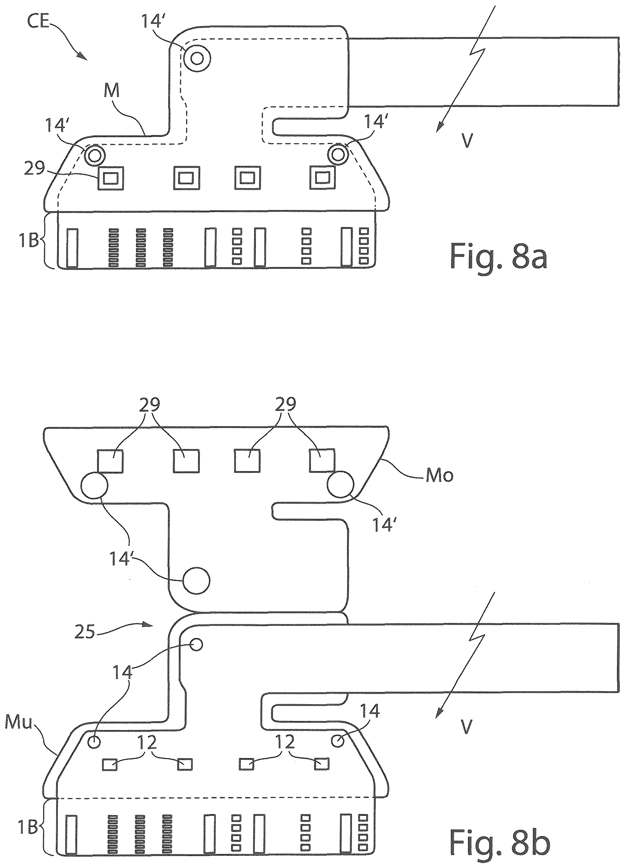

In den Figuren 8a bis f ist eine das Trägersubstrat 1 der implantierbaren Manschettenelektrode CE teilweise umfassende Manschette M dargestellt, die jenen Bereich des Trägersubstrates 1 sowohl an dessen Unter- sowie auch Oberseite umfasst, der sich unmittelbar an den Trägersubstratbereich 1B anschließt und sich im Unterschied zum Trägersubstratbereich 1B nicht im Wege einer materialinhärenten mechanischen Vorspannung selbständig geradzylinderförmig verformt und auf diese Weise bündig an das Epineurium des Nervenfaserbündels im implantierten Zustand in Anlage gebracht wird.

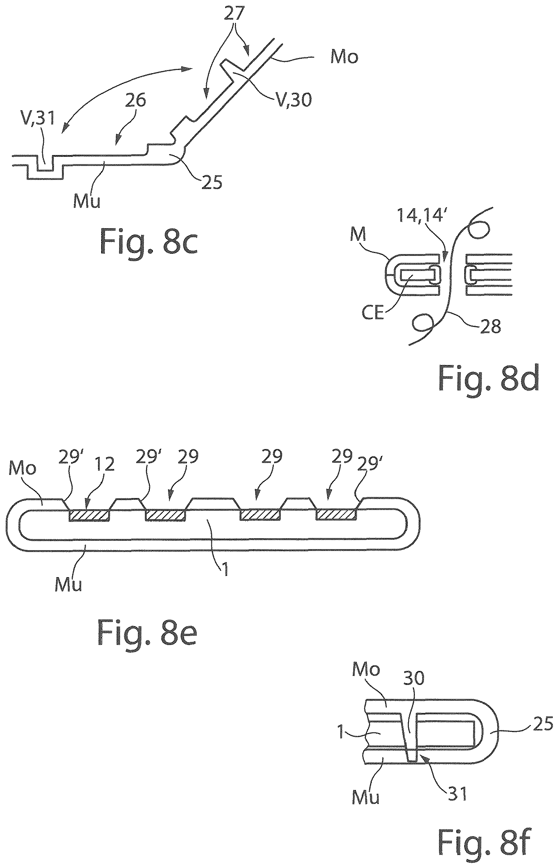

Die Manschette M dient in erster Linie einem verbesserten Handling der implantierbaren Manschettenelektrode CE, die aufgrund ihrer sehr geringen Trägersubstratdicke sowie der auf die Trägersubstratoberfläche aufgebrachten filigranen Elektrodenanordnungen vom Operateur eine besonders sorgfältige Handhabung abverlangt. Die Manschette M ist vorzugsweise einteilig ausgebildet und verfügt über ein Manschettenunterteil Mu sowie ein Manschettenoberteil Mo, die beide über ein Filmscharniergelenk 25 gelenkig verbunden sind, siehe hierzu Figuren 8b und 8c. Das Manschettenunterteil Mu verfügt über eine das Trägersubstrat 1 einbettende Vertiefung 26, in die das Trägersubstrat 1 einsetzbar ist. Im eingesetzten Zustand umfasst das Manschettenunterteil Mu das Trägersubstrat 1 in der aus Figur 8b entnehmbaren, umrahmenden Weise, das heißt das Manschettenunterteil MU ragt seitlich unter dem Trägersubstrat 1 hervor.

Das einstückig mit dem Manschettenunterteil Mu über das Scharniergelenk 25 verbundene Manschettenoberteil Mo ist in Form und Größe an das Manschettenunterteil Mu angepasst und verfügt gleichfalls wie das Manschettenunterteil Mu über eine das Trägersubstrat 1 einbettende Vertiefung 27, so dass im geschlossenem Zustand der Manschette M das Trägersubstrat 1 in der in Figur 8a dargestellten Weise hermetisch umfasst, wobei lediglich der Trägersubstratbereich 1B aus der Manschette M emporragt.

Neben einem verbesserten Handling dient die Manschette M insbesondere auch einer verbesserten Fixierung der Manschettenelektrode CE relativ zum Nervenfaserbündel. Hierzu sehen die Manschettenober- und -unterseiten Mo, Mu jeweils Befestigungsöffnungen 14' vor, siehe Fig. 8a, b, d, die deckungsgleich mit den innerhalb des Trägersubstrates 1 eingebrachten Befestigungsöffnungen 14 im zusammengeklappten Zustand der Manschette M sind. Auf diese Weise ist es möglich einen chirurgischen Faden 28 durch die Öffnungen 14, 14' der von der Manschette M umfassten Manschettenelektrode CE zu führen. Hierdurch kann die Metallring ummantelte Befestigungsöffnung 14 der Manschettenelektrode CE durch die innerhalb der Manschette M eingebrachte Befestigungsöffnung 14' entlastet werden. Vorzugsweise ist die Manschette M aus einem stabilen Kunststoffmaterial gefertigt, beispielsweise aus Parylen. Zur weiteren Steigerung der Festigkeit, kann die Mo und Mu auch aus einem Polymerhybrid bestehen (z.B. Parylen (innen) und Silikongummi aussen). Dieser Hybrid hat den Vorteil, dass die Stabilität des Parylen mit der Reißfestigkeit des Silikons kombiniert wird. In einer bevorzugten Ausführungsform sind die Befestigungsöffnungen 14' innerhalb der Manschette M durch entsprechende Materialaufdickung verstärkt ausgeführt.

In Manschettenoberteil Mo sind Öffnungsfenster 29 eingebracht, die einen freien Zugang zu den Referenzelektrodenflächen 12 gewährleisten. In Figur 8e ist ein diesbezüglicher Querschnitt durch das von der Manschette M umfasste Trägersubstrat 1 dargestellt, an dessen Oberseite Referenzelektrodenflächen 12 eingebracht sind, die durch die innerhalb des Manschettenoberteils Mo eingebrachten Öffnungsfenster 29 frei zugänglich bleiben. Vorzugsweise umfassen die Öffnungsfenster 29 die Referenzelektrodenflächen 12 mit einer schräg abfallenden Begrenzungsflanke 29', so dass dafür gesorgt ist, dass die Referenzelektrodenflächen 29 ganzflächig mit umliegendem Gewebe in Körperkontakt treten können.

Um sicherzustellen, dass die Manschette M in einem geschlossenen Zustand verbleibt, sind zwischen dem Manschettenober- und -unterteil Mo, Mu Verriegelungsstrukturen V angeordnet, die beispielsweise aus einem Pin 30 und einer gegenliegend angeordneten Vertiefung 31 bestehen, siehe Figuren 8c und f. Beim Zusammenklappen des Manschettenober- und -unterteils fügen sich die Pins 30 kraftbeaufschlagt in die entsprechenden Vertiefung 31, in der der Pin 31 jeweils reibschlüssig, dauerhaft gehalten wird. In Figur 48 ist der geschlossene Zustand einer Verriegelungsstruktur V illustriert. Hierbei ragt der am Manschettenoberteil Mo angebrachte Pin 30 durch eine entsprechende im Trägersubstrat 1 eingebrachte Öffnung und mündet endseitig innerhalb der Vertiefung 31 des Manschettenunterteils Mu. Selbstverständlich sind alternative Ausgestaltungsformen für die Verriegelungsstrukturen denkbar, beispielsweise in Form geeignet ausgebildeter Rastmechanismen.

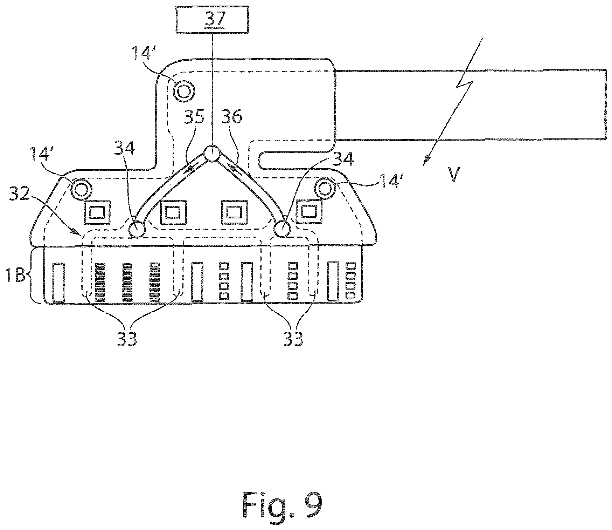

In Figur 9 ist eine weitere Ausführungsform illustriert, die eine erleichterte Implantation der lösungsgemäß ausgebildeten Manschettenelektrode CE ermöglicht. Innerhalb des Trägersubstrats 1 ist ein Fluidkanalsystem 32 eingearbeitet, das vollständig vom Trägersubstrat 1 umfasst ist. Das Fluidkanalsystem 32 erstreckt sich im Wesentlichen im Bereich des Trägersubstratbereiches 1B, der aufgrund einer materialinhärenten Vorspannung ohne äußere Krafteinwirkung im Wege einer autonomen Selbstaufwicklung die Gestalt eines Gradzylinders annimmt. Wird hingegen das Fluidkanalsystem 32 mit einem Fluid, vorzugsweise Wasser gefüllt, so vermag der sich längs des Fluidkanalsystems 32 ausbildende Wasserdruck den Trägersubstratbereich 1b entgegen der materialinhärenten Wickelkräfte eben aufzuspannen. Hierzu verfügt das Fluidkanalsystem 32 über sich in Umfangsrichtung der Mantelfläche des sich selbständig ausbildenden Gradzylinders verlaufende Fluidkanaläste 33, die im befüllten Zustand die erforderliche Streckung des Trägersubstratbereiches 1B erzwingen.

Zur Befüllung des Fluidkanalsystems 32 sind wenigstens zwei Kanalöffnungen 34 innerhalb des Trägersubstrats 1 vorgesehen, deren Größe und Anordnung derart bemessen sind, dass sie fluiddicht an Ein- und Austrittsöffnungen von innerhalb der Manschette M verlaufenden Fluidzu- bzw. -ableitungen 35, 36 münden. Die innerhalb der Manschette M verlaufenden Zu- bzw. Ableitungen 35, 36 sind fluidisch mit einem Fluidsteuersystem 37 verbunden, das von einem Operateur betätigbar ist.

Im Falle einer Implantation ist das Fluidkanalsystem 32 mit einem Fluid befüllt, wodurch der Trägersubstratbereich 1B gestreckt ist. In diesem Zustand platziert der Operateur die Manschettenelektrode CE exakt an einer vorgegebenen Stelle längs des Nervenbündels. Anschließend erfolgt die Entleerung des Fluidkanalsystems 32 durch den Operateur, wodurch sich der Trägersubstratbereich 1B selbsttätig um das Nervenfaserbündel wickelt. Als letzter Schritt wird die Manschettenelektrode CE mit einem chirurgischen Faden durch die in der Manschette vorgesehenen Befestigungsöffnungen 14' am umliegenden Gewebe fixiert.

In einer vorteilhaften Ausgestaltung des vorstehenden Fluidkanalsystems 32 ist es denkbar dieses mit einem Formgedächtnismetall- Formgedächtnispolymer zu füllen. Zu Zwecken der Aktivierung sind die Kanalöffnungen 34 mit metallisierten Kontakten versehen, über die eine elektrische Spannung längs der Zuleitungen 35, 36 zur Entfaltung der implantierbaren Elektrodenanordnung CE über ein entsprechend modifiziertes Steuergerät 37 anlegbar ist, bis die Elektrode letztlich platziert ist.

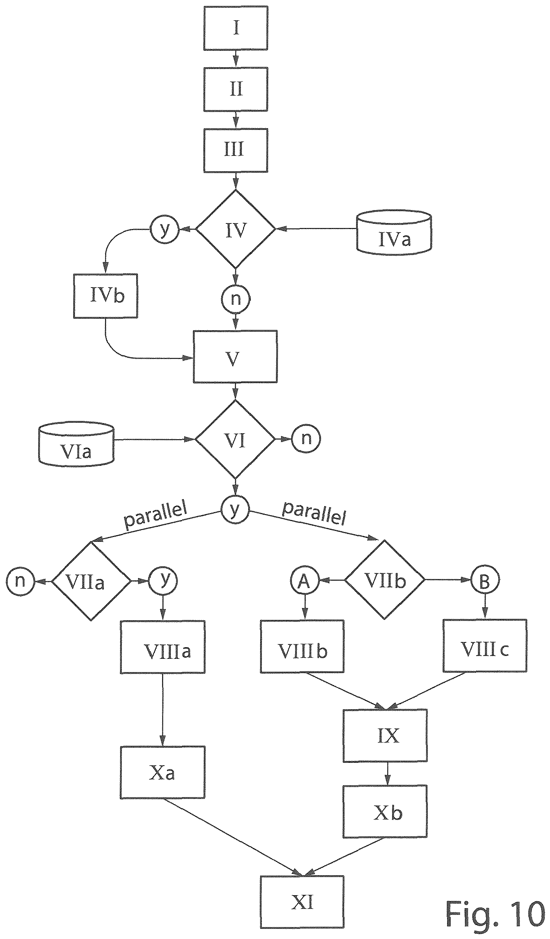

In Figur 10 ist ein Flussdiagramm illustriert, aus dem die Abfolge von Einzelschrittmaßnahmen für eine Blutdruckbeeinflussung bzw. -regulierung durch selektive elektrische Stimulation von Nervenfasern entnommen werden kann. Es sei angenommen, dass zu Zwecken der Stimulation, die in Figur 6 illustrierte Elektrodenanordnung lokal um den Vagusnerv appliziert ist, mit der die Inhibitionsfunktion möglich ist. Sollte auf eine Inhibition verzichtet werden können, so eignet sich gleichsam die Elektrodenanordnung gemäß Figur 2a. Die weitere Erläuterung nimmt zugleich Bezug auf die in Figur 1 dargestellten Komponenten der lösungsgemäßen implantierbaren Anordnung E. Um Wiederholungen zu vermeiden sei vorausgeschickt, dass die mit y bezeichneten Entscheidungspunkte einem "ja" und die mit n bezeichneten Entscheidungspunkte einem "nein" entsprechen.
I) Start: Aktivierung der implantierbaren Anordnung E entweder manuell oder automatisch, hierzu wird die Auswerte-/Steuereinheit in Form eines Microcontrollers initiiert (A/S, Fig.1)
II) Erfassung des EKG Signals mittels der Elektroden 12 der Manschettenelektrode E, siehe Fig. 6. Die Auswerte-/Steuereinheit A/S validiert hierbei die R-Zacke und separiert etwaige EMP Fehler aus dem Signal, wobei mehrere Durchläufe monitoriert werden. Die Auswerte-/Steuereinheit A/S bestimmt hierbei die Zuverlässigkeit, mit der die R-Zacken erkannt werden. Schließlich ermittelt die Auswerte-/Steuereinheit A/S die Herzschlagrate.
III) Erfassung des Blutdrucksignals SN mittels der ersten Elektrodenflächen 4, der ersten Elektrodenstreifen 5 sowie der EKG- Elektrodenflächen 12, der Manschettenelektrode E. Dies erfolgt durch kohärentes Mitteln der Signale der mittleren Reihe der ersten Elektrodenflächen 4, getriggert durch die Anstiegsflanke des zuvor ermittelten EKG Signales.
IV) Entscheiden ob Blutdruckänderung vorliegt.
IVa) Hierbei erfragt die Auswerte-/Steuereinheit A/S einen aktuellen ReferenzBlutdruck (SB) und vergleicht bzw. kalibriert die Amplitude des Referenzsignales mit dem Blutdrucksignals SN
IVb) Validierung des Blutdruckes und der Stimulationsposition, falls Blutdruckänderung vorliegt, siehe y. Die Auswerte-/Steuereinheit A/S fragt die Look up Tabelle LT nach bereits für diesen Patienten abgelegten Blutdrucksignalwerten SN sowie Zeit-Intervallen ZV und ZV* ab und vergleicht diese mit dem im Wege der Mittelung erfassten Blutdrucksignal SN. Die Auswerte-/Steuereinheit A/S ermittelt das "beste" SN einer Elektrode, diese wird als kommende Stimulationselektrode im Arbeitsspeicher markiert
V) Bestimmung des zeitlichen Versatzes ZV zwischen EKG-Signal und Blutdruckreferenzsignal. Hierbei ermittelt die Komparatoreinheit K1 den zeitlichen Versatz ZV zwischen R-Zacke und Anstiegsschwellwert und Referenzblutdruck.
   Die Komparatoreinheit K2 ermittelt den zeitlichen Versatz ZV* zwischen dem Anstiegsschwellwert des Referenzsignales und dem über die Elektrode erfassten neuronalen Blutdrucksignal siehe ZS in Fig. 3 und SN in Fig. 4. Aus dem Referenzblutdruck werden Beginn P1 und Ende P2 der Pulswelle PW bestimmt. Dieses Intervall ergibt T1, siehe auch Figur 3B und C. Das Intervall T1 wird um den zeitlichen Versatz ZV + ZV* versetzt und ergibt das Intervall ZF. (Siehe Fig. 3)
VI) Entscheidung zur Stimulation und Auswahl der Stimulationsparameter:
   Die Auswerte-/Steuereinheit A/S ermittelt die Uhrzeit UH und die aktuelle Lage und Bewegung des Patienten mit dem Beschleunigungssensor (BS).
   Die Auswerte-/Steuereinheit A/S ermittelt ferner die Impedanzen der Stimulationselektrode über den Inter-Stimulus Impedanz Detektor. Basierend auf den erhobenen Blutdruckwerten, der Herzschlagrate, der Aktivität des Patienten und dem Ausbleiben eines anderslautenden Steuerkommandos, z.B. ausgelöst durch externes Signal durch Radio Com Modul (SES), ein Fehlfunktionssignal einer Komponente des Implantates (z.B. eines IC der Stimulationsseite), oder der Detektion eines starken statischen Magnetfeldes (EMP), etc, entscheidet die Auswerte-/Steuereinheit A/S, ob eine Stimulation erfolgen soll, ja (y) oder nein (n).
VIa) Stimulationsreferenzwerte. Die Auswerte-/Steuereinheit A/S vergleicht die erhobenen Parameter mit bereits gespeicherten in der Look up Tabelle LT und dem Speichermodul SM und wählt passende Stimulationsparameter aus (passend bedeutet wie "stark" und wie "lange" muss stimuliert werden um den Blutdruck um x% zu senken).
   Die Stimulationskoordinaten wie (ZF, Anzahl und Form der Pulse werden dem "aktivierenden" Funktionsgenerator F1 (binär) mitgeteilt. Falls parallel selektiv Inhibiert werden muss, werden die passenden Stimulationsparameter für die selektive Inhibition an den Funktionsgenerator F2 (binär) weitergegeben.
Vlla) Die Auswerte-/Steuereinheit A/S entscheidet über das Inhibitionsverfahren (HF oder anodaler Block)
Vllb) Die Auswerte-/Steuereinheit A/S entscheidet über den Stimulationsmodus A oder B (siehe Fig. 5)
Vlllb) Vorbereitung/Modulation der aktivierenden Stimulationsparameter gemäß Modus A:
   Wird ein A-Modus für eine schnelle Intervention gewählt, wird ein fester Stimulationssweep mit definierter Anzahl an Einzelpulsen vorbereitet (Dauer ist nicht korreliert mit dem ZF Interval, sondern wird einer Tabelle entnommen), der mit vordefinierten Pausen wiederholt wird (Siehe Fig. 5 Modus A).
   Die Auswerte-/Steuereinheit A/S übergibt ein Template an den ersten Funktionsgenerator F1, der das Spannungssignal generiert, das an den Modulator M1 übertragen wird.
VIIIc) Vorbereitung/Modulation der aktivierenden Stimulationsparameter gemäß Modus B:
   Wird ein B-Modus gewählt, müssen die Einzelpulse weiter optimiert werden. Der erste Funktionsgenerator F1 erstellt ein analoges Template eines Stimulationsintervals (SSI) und passt eine definierte Anzahl an einzelnen biphasischen Pulsen in das Interval ZF ein. Hierbei wird das Stimulationssignal dem biologischen Signal angepasst. Der Referenzblutdruckverlauf wird als umhüllende Funktion über die Amplitude des Einzelpulse gelegt (siehe Fig. 4A). Der erste Funktionsgenerator F1 übergibt die Tabelle mit dem vorbereiteten Stimulationssweep an den Modulator M1.
IX) Anpassung der Phasen der Einzelpulse des Sweeps:
   Der Modulator M1 ist für beide Modi zuständig und variiert die beiden Phasen jedes einzelnen Pulses (siehe Fig. 4 B) um eine für den individuellen Patienten ideale Einzelpulsform zu erstellen. Der Modulator M1 übergibt das "fertige" Spannungssignal in Form eines Sweeps an den Signal-Strom-Umwandler (SSW1).
Xb) Durchführung der aktivierenden Stimulation:
   Der Signal-Strom-Umwandler SSW1 wartet auf das EKG Trigger Signal und wartet bis das "aktive Fenster für die Stimulation ZF" erreicht ist und überträgt den Stimulationssweep auf die zuvor ausgewählte Stimulationselektrode. Zwischen jedem Einzelpuls wird die Impedanz des Stimulationskontaktes durch die Elektrodenimpedanzmesseinheit EM erfasst. Wird eine Polarisation vermittels der Auswerte-/Steuereinheit A/S detektiert gibt diese dem aktiven Polarisationskompensator EE1 den Befehl zwischen jedem Puls eine kleine Extraladung zur Kompensation durch den Stimulationskontakt zu leiten. Reicht diese Interstimulationskompensation nicht aus, wird zudem der nach Beendigung des Sweeps der Intersweep-Kompensator aktiviert und gleicht eine Polarisation aus.
VIIIa) Vorbereitung/Modulation der inhibierenden Stimulationsparameter:
   Die Auswerte-/Steuereinheit übergibt dem Funktionsgenerator F2 ein Stimulationsintervall für die Inhibition. In aller Regel ist dies länger als die aktivierende Stimulation, d.h. es beginnt kurz davor und endet nach der aktivierenden Stimulation.
   Die Auswerte-/Steuereinheit A/S legt fest, ob der zweite Funktionsgenerator F2 einen anodalen Block legt, d.h. nur monophasischen Block oder ob ein HF-Block erfolgen soll. Der Funktionsgenerator 2 erstellt zudem entsprechend ein Stimulations(Spannungs-)Template. Im Falle eines HF-Blockes übergibt der F2 das Spannungssignal dem Modulator M2, um die einzelnen Phasen zu "glätten".
Xa) Durchführung der inhibierenden Stimulation:
   Der Signal-Strom-Umwandler SSW2 setzt das Signal entweder als anodaler Block des F2 oder als HF-Block des M2 in ein Stromsignal um und leitet es über die Inhibitionselektrode 9 der Reihe 13, siehe Figur 6. Die Elektrodenimpedanzmesseinheit EM überwacht die Polarisierung der inhibierenden Elektroden 9 und aktiviert über die Auswerte-/Steuereinheit A/S im Bedarfsfall die Entpolarisierungseinheit EE2 zur Kompensation.
   Beim HF-Block kann beides erfolgen interstimulus und intersweep, beim anodalen Block wird nur der Intersweepkomensator aktiv.
XI) Auswertung der Stimulation:
   Die Auswerte-/Steuereinheit A/S ermittelt die Veränderung der Blutdruckkurve und leitet eine Wiederholung ein. Im Falle des B-Modus kann als primärer Stimulationsparameter variiert werden wieviele Herzschläge die Stimulation pausiert. Über diese Funktion erfolgt die (patientenpezifische) Rückkopplung der Wirkungsweise des Implantates.
   Der Erfolg der Stimulation wird in einen Speicher geschrieben um für spätere Vergleiche benutzt werden zu können.

**Bezugszeichenliste**

| | |
|---|---|
| 1 | Trägersubstrat |
| 1' | Trägersubstratoberfläche |
| 1B | Trägersubstratbereich |
| 2 | erste Elektrodenanordnung |
| 3 | erste Elektrodenstrukturen |
| 4 | erste Elektrodenflächen |
| 4a | axiale Erstreckung der ersten Elektrodenflächen |
| 4U | in Umfangsrichtung orientierte Erstreckung der ersten Elektrodenflächen |
| 5 | erste Elektrodenstreifen |
| 6, 6' | Signaldetektor und -generator |
| 7 | dritte Elektrodenanordnung |
| 8 | dritte Elektrodenstreifen |
| 9 | dritte Elektrodenflächen |
| 9a | axiale Erstreckung der dritten Elektrodenflächen |
| 9U | in Umfangsrichtung orientierte Erstreckung der dritten Elektrodenflächen |
| 10 | Lichtwellenleiteranordnung |
| 11 | Lichtwellenleiteröffnungen |
| 12 | Zweite Elektrodenanordnung, EKG-Elektrodenflächen |
| 13 | dritte Elektrodenstruktur |
| 14 | Befestigungsöffnungen |
| 15 | Öffnung |
| 16 | Elektrodenstreifenoberfläche |
| 17 | Basisplatte |
| 18 | Oberseite |
| 19 | Unterseite |
| 20 | Strukturelement |
| 21 | Oberflächen bereich |
| 22 | Haftvermittlerschicht |
| 22' | Haftvermittlerschichtanordnung |
| 23 | Zweiter Oberflächenbereich |
| 24 | dritter Oberflächenbereich |
| A | Axiale Richtung |
| A/S | Auswerte-/Steuereinheit |
| A3, A4 | Amplitude |
| Amax | Maximale Amplitude |
| AT | Anodischer Signalteil |
| BS | Beschleunigungssensor |
| DE | Delle |
| E | Implantierbare Elektrodenanordnung, Manschettenelektrode |
| E1 | Anodische Amplitude |
| E2 | Kathodische Amplitude |
| E3 | Kathodische Pulsbreite |
| E4 | Anodische Amplitude |
| E5 | Wiederholrate |
| E6 | Repolarisationsflanke |
| E7 | Pause, Nullpegel zwischen anodischer und kathodischer Amplitude |
| EE1, EE2 | Entpolarisierungseinheit |
| EKG | EKG-Zeitsignal |
| EM | Elektrodenimpedanzmesseinheit |
| EMP | Schutzeinheit gegenüber Elektromagnetischer Pulseinwirkung EMP/MRT |
| EP | Einzelpuls |
| ES | Energiespeicher / Energiequelle |
| F1, F2 | Funktionsgenerator |
| G | Gehirn |
| H | Herz |
| KO | Geometrische Konstellation |
| KT | Kathodischer Signalteil |
| L | Leiterbahn |
| LA | Längsachse des Strukturelementes |
| LI | Lichtwellenleiter |
| LQ | Lichtquelle(n) |
| LT | Look/up - Tabelle |
| M | Maximum |
| M1, M2 | Modulator |
| NF | Nervenfaser |
| NFB | Nervenfaserbündel |
| P1, P2 | Charakteristische Phasenpunkte längs des Zeitsignals |
| PW | Pulswelle, Blutdruckwelle |
| R | R-Zacke des EKG-Signals |
| SB | Blutdrucksensor |
| SES | Signal- und Energieversorgungseinheit |
| SM | Speicher Modul |
| SN | Natürliches neuronales elektrisches Signal |
| SSI | Stimulationssignal |
| SSW1, SSW2 | Signal-Strom Wandler |
| T | Timereinheit |
| T1 | Zeitdauer der Pulswelle |
| T_{SN} | Pulsdauer eines natürlichen neuronalen, elektrischen Signals |
| U | Umfangsrichtung |
| UH | Uhr |
| V | Verbindungsstruktur |
| ZF | Zeitfenster |
| ZS | Zeitsignal |
| ZV, ZV* | Zeitversatz |

## Patentansprüche

1. Implantierbare Anordnung zur ortsselektiven Erfassung neuronaler elektrischer Signale, die sich längs wenigstens einer in einem Nervenfaserbündel enthaltenden Nervenfaser ausbreiten, sowie zur selektiven elektrischen Stimulation der wenigstens einen Nervenfaser, umfassend folgende Komponenten:
- Implantierbare Elektrodenanordnung (E), die auf einem um das Nervenfaserbündel manschettenartig anlegbaren biokompatiblen Trägersubstrat (1) angeordnet ist, das eine im implantierten Zustand dem Nervenfaserbündel zugewandt orientierte, geradzylinderförmige Trägersubstratoberfläche (1') aufweist, an der eine erste Elektrodenanordnung (2) zur ortsselektiven Erfassung der neuronalen elektrischen Signale und selektiven elektrischen Stimulation der wenigstens einen Nervenfaser, und an dem eine zweite Elektrodenanordnung (12) zur Erfassung eines EKG-Signals angeordnet ist,
- eine mit der implantierbaren Elektrodenanordnung (E) elektrisch leitend verbindbare oder verbundene Auswerte-/Steuereinheit (A/S), in der die ortsselektiv erfassten, neuronalen, elektrischen Signale sowie das EKG-Signal zeitaufgelöst derart auswertbar sind, dass ein zu einem physiologischen Parameter, vorzugsweise Blutdruck, korreliertes neuronales Zeitsignal ableitbar ist,
- eine mit der Auswerte-/Steuereinheit (A/S) verbundene erste Komparatoreinheit (K1), in der ein charakteristischer relativer Zeitversatz zwischen dem EKG-Signal und dem mit dem physiologischen Parameter korrelierten, neuronalen Zeitsignal ermittelbar ist,
- ein mit der Auswerte-/Steuereinheit (A/S) verbundener erster Funktionsgenerator (F1), der innerhalb eines durch die Auswerte-/Steuereinheit (A/S) unter Zugrundelegung des relativen Zeitversatzes ermittelten Zeitfensters ein sich aus einer Vielzahl n Einzelpulsen (EP) zusammensetzendes und in Phase und zeitlichen Amplitudenverlauf an das abgeleitete, mit dem physiologischen Parameter korrelierte, neuronale Zeitsignal angepasstes Stimulationssignal generiert sowie
- einen mit dem ersten Funktionsgenerator (F1) und der ersten Elektrodenanordnung (2) mittel- oder unmittelbar verbundenen ersten Signal-Strom-Wandler (SSW1), der das Stimulationssignal zur selektiven elektrischen Stimulation der wenigstens einen Nervenfaser an die erste Elektrodenanordnung (2) leitet,
wobei die Auswerte-/Steuereinheit (A/S), die erste Komparatoreinheit (K1), der erste Funktionsgenerator (F1) sowie der erste Signal-Strom-Wandler (SSW1) als implantierbares Modul zusammengefasst sind.

2. Implantierbare Anordnung nach Anspruch 1,
**dadurch gekennzeichnet, dass** wenigstens eine zweite Komparatoreinheit (K2) mit der Auswerte-/Steuereinheit (A/S) verbunden ist,
dass die zweite Komparatoreinheit (K2) wenigstens einen Signalpegel, der dem mit dem physiologischen Parameter korrelierten, neuronalen Zeitsignal zugeordnet ist, mit einem Referenzsignal vergleicht und einen Differenzpegelwert generiert, und dass die Auswerte-/Steuereinheit (A/S) wenigstens auf Grundlage des Differenzpegelwertes wenigstens den zeitlichen Amplitudenverlauf des Stimulationssignals festlegt.

3. Implantierbare Anordnung nach Anspruch 1 oder 2,
**dadurch gekennzeichnet, dass** die implantierbare Elektrodenanordnung (E), an der im implantierten Zustand, dem Nervenfaserbündel zugewandt orientierten, geradzylinderförmigen Trägersubstratoberfläche (1') eine dritte Elektrodenanordnung (7) zur Inhibition von sich unidirektional längs des Nervenfaserbündels ausbreitenden neuronalen elektrischen Signalen umfasst.

4. Implantierbare Anordnung nach einem der Ansprüche 1 bis 3,
**dadurch gekennzeichnet, dass** das implantierbare Modul über einen elektrischen Energiespeicher (ES) und/oder eine Induktionsbasierte Signal- und Energieversorgungseinheit (SES) verfügt, die zumindest mit der Auswerte-/Steuereinheit (A/S) elektrisch verbunden ist.

5. Implantierbare Anordnung nach einem der Ansprüche 1 bis 4,
**dadurch gekennzeichnet, dass** in dem implantierbaren Modul ein Beschleunigungssensor (BS) integriert ist und/oder ein zum Modul separater, implantierbar ausgebildeter Beschleunigungssensor (BS') vorgesehen ist, und
dass der Beschleunigungssensor (BS, BS') Beschleunigungsinformationen generiert und mit der Auswerte-/Steuereinheit (A/S) elektrisch verbunden ist.

6. Implantierbare Anordnung nach einem der Ansprüche 1 bis 5,
**dadurch gekennzeichnet, dass** der erste Funktionsgenerator (F1) zur Erzeugung von Einzelpulsen in der Lage ist, die jeweils aus einem polarisierenden und einem repolarisierenden rechteckpulsförmigen Teilsignal zusammengesetzt sind, und dass zwischen dem ersten Funktionsgenerator (F1) und dem ersten Signal-Strom-Wandler (SSW1) ein erster Modulator (M1) geschaltet ist, der einen dem rechteckpulsförmigen, repolarisierenden Teilsignal zugeordneten Signalflankenverlauf zeitlich gegenüber dem polarisierenden Teilsignal vergrößert und glättet und eine jeweils beiden Teilsignalen zugeordnete Signalstärke vereinheitlicht.

7. Implantierbare Anordnung nach einem der Ansprüche 3 bis 6,
**dadurch gekennzeichnet, dass** ein mit der Auswerte-/Steuereinheit (A/S) verbundener zweiter Funktionsgenerator (F2) vorgesehen ist, der zeitlich vor und/oder während des ermittelten Zeitfensters ein elektrisches Blockierungssignal generiert, und
dass der zweite Funktionsgenerator (F2) mittel- oder unmittelbar mit einem zweiten Signal-Strom-Wandler (SSW2) verbunden ist, der das elektrische Blockierungssignal der dritten Elektrodenanordnung (7) zuführt.

8. Implantierbare Anordnung nach Anspruch 7,
**dadurch gekennzeichnet, dass** der zweite Funktionsgenerator (F2) zur Erzeugung von Einzelpulsen in der Lage ist, die jeweils aus einem polarisierenden und einem repolarisierenden rechteckpulsförmigen Teilsignal zusammengesetzt sind, und
dass zwischen dem zweiten Funktionsgenerator (F2) und dem zweiten Signal-Strom-Wandler (SSW2) ein zweiter Modulator (M2) geschaltet ist, der einen dem rechteckpulsförmigen, repolarisierenden Teilsignal zugeordneten Signalflankenverlauf zeitlich gegenüber dem polarisierenden Teilsignal vergrößert und glättet und eine jeweils beiden Teilsignalen zugeordnete Signalstärke vereinheitlicht.

9. Implantierbare Anordnung nach Anspruch 8,
**dadurch gekennzeichnet, dass** der zweite Funktionsgenerator (F2) sowie das zweite Modul (M2) in dem implantierbaren Modul integriert sind.

10. Implantierbare Anordnung nach Anspruch 6 oder 8,
**dadurch gekennzeichnet, dass** der erste oder zweite Modulator (M1, M2) derart ausgebildet ist, dass zu jedem Einzelimpuls das polarisierende Teilsignal zeitlich durch einen durch den ersten Modulator (M1) erzeugbaren Nullsignalpegel vom repolarisierenden Teilsignal trennbar ist.

11. Implantierbare Anordnung nach einem der Ansprüche 1 bis 10,
**dadurch gekennzeichnet, dass** eine Elektrodenimpedanzmesseinheit (EM) Teil des implantierbaren Moduls ist oder an dem Trägersubstrat (1) angeordnet ist und mit der Elektrodenanordnung (E) sowie mit der Auswerte-/Steuereinheit (A/S) verbunden ist, und dass die Elektrodenimpedanzmesseinheit (EM) zwischen jedem der n Einzelpulse eine Impedanzmessung zumindest an Elektroden der ersten Elektrodenanordnung (2) vornimmt.

12. Implantierbare Anordnung nach Anspruch 11,
**dadurch gekennzeichnet, dass** eine elektrische Entpolarisierungseinrichtung (EE) Teil des implantierbaren Moduls ist oder an dem Trägersubstrat (1) angeordnet ist und mit der Elektrodenanordnung (E) sowie mit der Auswerte-/Steuereinheit (A/S) verbunden ist, und
dass die Entpolarisierungseinrichtung (EE) im Falle einer durch die Elektrodenimpedanzmesseinheit (EM) festgestellten Restpolarisation an einzelnen Elektroden diese selektiv mittels elektrischer Signalbeaufschlagung entpolarisiert.

13. Implantierbare Anordnung nach einem der Ansprüche 4 bis 12,
**dadurch gekennzeichnet, dass** ein separates Blutdruckmesssystem (SB) vorgesehen ist, das absolute Blutdruckwerte erfasst, die über die Induktionsbasierte Signal- und Energieversorgungseinheit (SES) der Auswerte-/Steuereinheit (A/S) zuführbar sind, und dass die Auswerte-/Steuereinheit (A/S) auf der Grundlage der absoluten Blutdruckwerte, dem EKG-Signal, dem mit dem physiologischen Parameter korrelierten, neuronalen Zeitsignal sowie wenigstens einem Referenzsignal den ersten Funktionsgenerator (F1) zur Generierung des Stimulationssignal ansteuert.

14. Implantierbare Anordnung nach Anspruch 2, 5 und 13,
**dadurch gekennzeichnet, dass** eine Speichereinheit, vorzugsweise in Art einer Look-up-Tabelle, vorgesehen ist, die Teil der Auswerte-/Steuereinheit (A/S) oder als separate Einheit mit dieser verbunden ist,
dass in der Speichereinheit wenigstens eine der folgenden Informationen abspeicherbar und abrufbar sind: Differenzpegelwert, Beschleunigungsinformationen, erfasster absoluter Blutdruck.

15. Implantierbare Anordnung nach einem der Ansprüche 1 bis 14,
**dadurch gekennzeichnet, dass** die dem Nervenfaserbündel zugewandt orientierte, geradzylinderförmige Trägersubstratoberfläche (1') eine axiale (a) sowie eine in Umfangsrichtung (U) orientierte Erstreckung besitzt und an der die erste Elektrodenanordnung (2) angebracht ist, die
- in axialer Abfolge wenigstens drei erste Elektrodenstrukturen (3) mit jeweils wenigstens zwei in Umfangsrichtung angeordneten ersten Elektrodenflächen (4), sowie
- wenigstens zwei axial zueinander beabstandete, sich in Umfangsrichtung erstreckende und jeweils eine Ringform annehmende erste Elektrodenstreifen (5) umfasst, die die wenigstens drei Elektrodenstrukturen axial beidseitig einschließen, und mit der Auswerte-/Steuereinheit (A/S) verbindbar oder verbunden ist.

16. Implantierbare Anordnung nach Anspruch 15 sowie einem der Ansprüche 3 bis 14,
**dadurch gekennzeichnet, dass** die dritte Elektrodenanordnung (7) in axialer Abfolge auf der dem Nervenfaserbündel zugewandten geradzylinderförmigen Trägersubstratoberfläche (1') axial neben der ersten Elektrodenanordnung (2) angeordnet ist und wenigstens zwei axial voneinander beabstandete, sich in Umfangsrichtung (U) erstreckende und jeweils eine Ringform annehmende dritte Elektrodenstreifen (8) sowie
axial zwischen den wenigstens zwei dritten Elektrodenstreifen (8) wenigstens eine, jeweils wenigstens zwei in Umfangsrichtung gleichverteilt angeordnete dritte Elektrodenflächen (9) umfassende dritte Elektrodenstruktur (13) umfasst.

17. Implantierbare Anordnung nach Anspruch 15 und 16,
**dadurch gekennzeichnet, dass** die ersten und dritten Elektrodenflächen (4, 9) in Umfangsrichtung (U) jeweils längs einer virtuellen Kreislinie gleichverteilt angeordnet sind.

18. Implantierbare Anordnung nach Anspruch 15 und 16,
**dadurch gekennzeichnet, dass** die ersten und dritten Elektrodenflächen (4, 9) jeweils eine axiale (4a, 9a) und eine in Umfangsrichtung (4U, 9U) orientierte Erstreckung aufweisen,
dass die Erstreckungen (4a, 4U) der ersten Elektrodenflächen (4) jeweils identisch sind,
dass die Erstreckungen (9a, 9U) der dritten Elektrodenflächen (9) jeweils identisch sind, und
dass die in Umfangsrichtung orientierte Erstreckung (9U) der dritten Elektrodenflächen (9) größer ist als die in Umfangsrichtung orientierte Erstreckung (4U) der ersten Elektrodenflächen (4).

19. Implantierbare Anordnung nach Anspruch 18,
**dadurch gekennzeichnet, dass** die axialen Erstreckungen (4a, 4U) der ersten und dritten Elektrodenflächen (4, 9) gleich sind.

20. Implantierbare Anordnung nach einem der Ansprüche 16 bis 19,
**dadurch gekennzeichnet, dass** die Trägersubstratoberfläche (1'), auf der die erste und dritte Elektrodenanordnung (2, 7) aufgebracht ist, einstückig zusammenhängend, d.h. unterbrechungsfrei, ausgebildet ist.

21. Implantierbare Anordnung nach einem der Ansprüche 16 bis 20,
**dadurch gekennzeichnet, dass** der axiale Abstand zwischen den ersten Elektrodenstreifen (5) größer oder gleich dem axialen Abstand der dritten Elektrodenstreifen (8) gewählt ist, und
dass der axiale Abstand zwischen den dritten Elektrodenstreifen zwischen 0,5 cm und 3 cm, vorzugsweise zwischen 0,75 cm und 1,25 cm misst.

22. Implantierbare Anordnung nach einem der Ansprüche 16 bis 21,
**dadurch gekennzeichnet, dass** die Form und Größe der ersten und dritten Elektrodenstreifen (5, 8) identisch sind,
dass die Flächengrößen jeweils der ersten und dritten Elektrodenfläche (4, 9) kleiner sind als die Flächengröße jeweils der ersten oder dritten Elektrodenstreifen (5, 8), vorzugsweise kleiner als ein Viertel der Flächengröße der ersten oder dritten Elektrodenstreifen (5, 8).

23. Implantierbare Anordnung nach einem der Ansprüche 16 bis 22,
**dadurch gekennzeichnet, dass** die ersten Elektrodenflächen (4) aus einem metallischen Material, vorzugsweises Iridium-Oxid, bestehen, das über eine höhere Ladungsübertragungskapazität verfügt als ein Material aus dem die dritten Elektrodenflächen (9) bestehen.

24. Implantierbare Anordnung nach Anspruch 23,
**dadurch gekennzeichnet, dass** das metallische Material der ersten Elektrodenflächen (4) Iridium-Oxid ist, und/oder
dass das Material der dritten Elektrodenflächen (9) ein metallisches Material, vorzugsweise Platin, oder ein elektrisch leitfähiges Polymer ist.

25. Implantierbare Anordnung nach einem der Ansprüche 16 bis 24,
**dadurch gekennzeichnet, dass** die erste und dritte Elektrodenanordnung (2, 7) jeweils als Tripolar-Elektrodenanordnung betreibbar ist, d.h. die ersten und dritten Elektrodenstreifen sind jeweils gegenpolig zur ersten und dritten Elektrodenstruktur (3, 13) polarisierbar.

26. Implantierbare Anordnung nach einem der Ansprüche 16 bis 25,
**dadurch gekennzeichnet, dass** die dritte Elektrodenanordnung (7) wenigstens eine optische Lichtwellenleiteranordnung (10) vorsieht, die wenigstens zwei in Umfangsrichtung (U) verteilt angeordnete, separate Lichtwellenleiteröffnungen (11) umfasst.

27. Implantierbare Anordnung nach Anspruch 26,
**dadurch gekennzeichnet, dass** die wenigstens zwei separaten Lichtwellenleiteröffnungen (11) längs einer virtuellen Kreislinie gleichverteilt angeordnet sind, und
dass die Lichtwellenleiteröffnungen (11) jeweils eine axiale (11a) und eine in Umfangsrichtung (11U) orientierte Erstreckung besitzen, die jener Erstreckungen (9a, 9U) der zweiten Elektrodenflächen (9) entsprechen.

28. Implantierbare Anordnung nach einem der Ansprüche 16 bis 27,
**dadurch gekennzeichnet, dass** die ersten Elektrodenflächen (4) sowie die ersten Elektrodenstreifen (5) der ersten Elektrodenanordnung (2) sowie die dritten Elektrodenflächen (9) sowie die dritten Elektrodenstreifen (8) der dritten Elektrodenanordnung (7) jeweils derart an der Trägersubstratoberfläche (1') angebracht sind, so dass sie die Trägersubstratoberfläche (1') nicht überragen.

29. Implantierbare Anordnung nach einem der Ansprüche 1 bis 28,
**dadurch gekennzeichnet, dass** das Trägersubstrat (1) aus wenigstens einem biokompatiblen Polymer gefertigt ist und zumindest bereichsweise an der dem Nervenfaserbündel zugewandten geradzylinderförmigen Trägersubstratoberfläche (1') einen Inflammationsreaktionen inhibierenden Wirkstoff aufweist.

30. Implantierbare Anordnung nach einem der Ansprüche 1 bis 29,
**dadurch gekennzeichnet, dass** das Trägersubstrat (1) biokompatibles Polymer enthält.

31. Implantierbare Anordnung nach Anspruch 30 sowie Anspruch 16,
**dadurch gekennzeichnet, dass** die ersten und/oder dritten Elektrodenstreifen (5, 8) jeweils über wenigstens eine lokale Öffnung verfügen, und dass die ersten und/oder dritten Elektrodenstreifen (5, 8) derart mit der Trägersubstratoberfläche (1') flächig verbunden sind, sodass das Polymer die wenigstens eine Öffnung wenigstens teilweise durchdringt.

32. Implantierbare Anordnung nach einem der Ansprüche 1 bis 31,
**dadurch gekennzeichnet, dass** rückseitig zur Trägersubstratoberfläche (1') an dem Trägersubstrat (1) wenigstens zwei Referenzelektrodenflächen (12) angebracht sind.

33. Implantierbare Anordnung nach einem der Ansprüche 1 bis 32,
**dadurch gekennzeichnet, dass** das biokompatible Trägersubstrat (1) im Bereich der dem Nervenfaserbündel zugewandt orientierten, geradzylinderförmigen Trägersubstratoberfläche (1') jeweils axial sich gegenüberliegende Randbereiche (14) aufweist, an denen das Trägersubstrat (1) über eine größere Substratdicke verfügt als im übrigen Trägersubstratbereich, und
dass die Randbereiche (14) über abgerundete Randkanten verfügen.

34. Implantierbare Anordnung nach einem der Ansprüche 1 bis 33,
**dadurch gekennzeichnet, dass** das biokompatible Trägersubstrat (1) in einem Trägersubstratbereich, der nicht um das Nervenfaserbündel manschettenartig anlegbar ist, wenigstens eine das Trägersubstrat vollständig durchsetzende Öffnung (15) aufweist.

35. Implantierbare Anordnung nach Anspruch 34,
**dadurch gekennzeichnet, dass** die wenigstens eine Öffnung (15) zumindest bereichsweise mit einem metallischen Material ummantelt ist.

36. Implantierbare Anordnung nach Anspruch 16 und 30,
**dadurch gekennzeichnet, dass** die ersten und/oder dritten Elektrodenstreifen (5, 8) jeweils ein metallisches Elektrodenstreifensubstrat mit einer ebenen Ober- und Unterseite aufweisen, mit wenigstens einem die Oberseite orthogonal, lokal überragenden Strukturelement,
dass die ebene Oberfläche des metallischen Elektrodenstreifensubstrats parallel zur Trägersubstratoberfläche orientiert ist, und
dass das Elektrodenstreifensubstrat vollständig von dem biokompatiblen Polymer umschlossen ist mit Ausnahme eines Oberflächenbereiches des wenigstens einen Strukturelements, der der Trägersubstratoberfläche zugewandt orientiert ist und diese nicht überragt.

37. Implantierbare Anordnung nach Anspruch 36,
**dadurch gekennzeichnet, dass** zumindest zwischen der Unterseite des Elektrodenstreifensubstrats und dem biokompatiblen Polymer des Trägersubstrats eine Haftvermittlerschicht oder Haftvermittlerschichtanordnung eingebracht ist.

38. Implantierbare Anordnung nach Anspruch 36 oder 37,
**dadurch gekennzeichnet, dass** der eine Oberflächenbereich des wenigstens einen Strukturelements oder eine dem Oberflächenbereich zugeordnete Ebene parallel zur Trägersubstratoberfläche orientiert, dass der Oberflächenbereich von Seiten der Trägersubstratoberfläche frei zugänglich angeordnet ist und dass das wenigstens eine Strukturelement einstückig mit den Elektrodenstreifensubstrats verbunden ist.

39. Implantierbare Anordnung nach einem der Ansprüche 36 bis 38,
**dadurch gekennzeichnet, dass** eine Vielzahl an der Oberseite des metallischen Elektrodenstreifensubstrats geometrisch gleichverteilt angeordnete und identisch ausgebildete Strukturelemente vorgesehen ist.

40. Implantierbare Anordnung nach einem der Ansprüche 36 bis 39,
**dadurch gekennzeichnet, dass** das wenigstens eine Strukturelement säulen-, rippen-, hülsen oder stegartig ausgebildet ist.

## Claims

1. An implantable device for the location-selective recording of neuronal electrical signals propagating along at least one nerve fibre contained in a nerve fibre bundle, as well as for the selective electrical stimulation of the at least one nerve fibre, comprising the following components:
- implantable electrode arrangement (E) which is arranged on a biocompatible carrier substrate (1) which can be applied in a cuff-like manner around the nerve fibre bundle and which in the implanted state has, orientated towards the nerve fibre bundle, a straight cylindrical carrier substrate surface (1') on which a first electrode arrangement (2) for the location-selective recording of the neuronal electrical signals and selective electrical stimulation of the at least one nerve fibre, and on which a second electrode arrangement (12) for recording an ECG signal are arranged,
- an evaluation/control unit (A/S), which can be or is connected in an electrically conducting manner to the implantable electrode arrangement (E), in which the location-selectively recorded, neuronal, electrical signals as well as the ECG signal are evaluated in a time-resolved manner in such a way that a neuronal time signal correlated to a physiological parameter, preferably blood pressure, can be derived,
- a first comparator unit (K1), connected to the evaluation/control unit (A/S), in which a characteristic relative time offset between the ECG signal and neuronal time signal correlated with the physiological parameter can be determined,
- a first function generator (F1), connected to the evaluation/control unit (A/S), which within a time window determined by the evaluation/control unit (A/S) on the basis of the relative time offset generates a stimulation signal, adapted in terms of phase and time amplitude to the derived neuronal time signal correlated to the physiological parameter and composed of a plurality n of individual pulses (EP) as well as
- a first signal-current converter (SSW1) which is directly or indirectly connected to the first function generator (F1) and the first electrode device (2) and sends the stimulation signal for the selective electrical stimulation of the at least one nerve fibre to the first electrode device (2),
wherein the evaluation/control unit (A/S), the first comparator unit (K1), the first function generator (F1) as well as the first signal-current converter (SSW1) are combined as an implantable module.

2. The implantable device according to claim 1,
**characterised in that** at least one second comparator unit (K2) is connected to the evaluation/ control unit (A/S),
**in that** the second comparator unit (K2) compares at least one signal level, which is assigned to the neuronal time signal correlated to the physiological parameter, with a reference signal and generates a difference level value, and
**in that** at least on the basis of the difference level value the evaluation/control unit (A/S) determines at least temporal amplitude profile of the simulation signal.

3. The implantable device according to claim 1 or 2,
**characterised in that** in the implanted state, on the straight cylindrical carrier substrate surface (1') orientated toward the nerve fibre bundle the implantable electrode arrangement (E) comprises a third electrode arrangement (7) for the inhibition of neuronal electrical signals propagating unidirectionally along the nerve fibre bundle.

4. The implantable device according to any one of claims 1 to 3,
**characterised in that** the implantable module comprises an electrical energy store (ES) and/or an induction-based signal and energy supply unit (SES) which is at least electrically connected to the evaluation/control unit (A/S).

5. The implantable device according to any one of claims 1 to 4,
**characterised in that** an acceleration sensor (BS) is integrated into the implantable module and/or an implantably designed acceleration module (BS') that is separate from the module is provided, and **in that** the acceleration sensor (BS, BS') generates acceleration information and is electrically connected to the evaluation/control unit (A/S).

6. The implantable device according to any one of claims 1 to 5,
**characterised in that** the first function generator (F1) is able to produce individual pulses which are each composed of a polarising and a repolarising square pulse-shaped partial signal, and **in that** between the first function generator (F1) and the first signal-current converter (SSW1) a first modulator (M1) is connected which enlarges and smooths a signal flank course assigned to the square pulse-shaped repolarising partial signal time-wise with regard to the polarising partial signal and harmonises a signal strength associated with each of the two partial signals.

7. The implantable device according to any one of claims 3 to 6,
**characterised in that** a second function generator (F2), connected to the evaluation/control unit (A/S), is provided, which in terms of time before and/or during the determined time window generates an electrical blocking signal and
**in that** the second function generator (F2) is directly or indirectly connected to a second signal-current converter (SSW2) which supplies the electrical blocking signal to the third electrode arrangement (7).

8. The implantable device according to claim 7,
**characterised in that** the second function generator (F2) is able to produce individual pulses which are each composed of a polarising and a repolarising square pulse-like partial signal and
**in that** between the second function generator (F2) and the second signal-current converter (SSW2) a second modulator (M2) is connected which enlarges and smooths a signal flank course assigned to the square pulse-shaped repolarising partial signal time-wise with regard to the polarising partial signal and harmonises a signal strength associated with each of the two partial signals.

9. The implantable device according to claim 8,
**characterised in that** the second function generator (F2) as well as the second module (M2) are integrated into the implantable module.

10. The implantable device according to claim 6 or 8,
**characterised in that** the first or second modulator (M1, M2) is designed in such a way that for each individual impulse the polarising partial signal can be separated from the repolarising partial signal by a zero signal level which can be produced by the first modulator (M1).

11. The implantable device according to any one of claims 1 to 10,
**characterised in that** an electrode impedance measuring unit (EM) is part of the implantable module or is arranged on the carrier substrate (1) and connected to the electrode arrangement (E) as well as to the evaluation/control unit (A/S) and **in that** between each of the n individual pulses the electrode impedance unit (EM) performs an impedance measurement at least on electrodes of the first electrode arrangement (2).

12. The implantable device according to claim 11, **characterised in that** an electrical depolarisation device (EE) is part of the implantable module or is arranged on the carrier substrate (1) and is connected to the electrode arrangement (E) as well as to the evaluation/control unit (A/S) and
**in that** in the event of residual polarisation on individual electrodes detected by the electrode impedance measuring unit (EM) the depolarisation device (EE) selectively depolarises these by means of applying electrical signals.

13. The implantable device according to any one of claims 4 to 12,
**characterised in that** a separate blood pressure measuring system (SB) is provided which records absolute blood pressure values which can be supplied via the induction-based signal and energy supply unit (SES) to the evaluation/control unit (A/S) and **in that** on the basis of the absolute blood pressure values, the ECG signal, the neuronal time signal correlated with the physiological parameter as well as at least one reference signal, the evaluation/control unit (A/S) actuates the first function generator (F1) to generate the stimulation signal.

14. The implantable device according to claim 2, 5 and 13,
**characterised in that** a storage unit, preferably in the form of a look-up table, is provided which is part of the evaluation/control unit (A/S) or is connected thereto as a separate unit,
**in that** in the storage unit at least one following pieces of information can be stored and called up;
differential level value, acceleration information, recorded absolute blood pressure.

15. The implantable device according to any one of claims 1 to 14,
**characterised in that** the straight cylindrical carrier substrate surface (1') orientated towards the nerve fibre bundle has an axial (a) as well as circumferentially (U) orientated first extent and on which the first electrode arrangement (2) is applied, which
- in axial sequence comprises at least three first electrode structures (3) each with at least two first electrode surfaces (4) arranged in the circumferential direction as well as
- at least two axially spaced first electrode strips (5) extending in the circumferential direction and each assuming an annular shape and which axially encompass the at least three electrode structures on both sides and is connected or connectable to the evaluation/control unit (A/S).

16. The implantable device according to claim 15 as well as any one of claims 3 to 14,
**characterised in that** the third electrode arrangement (7) is arranged in axial sequence on the straight-cylindrical support carrier surface (1') facing the nerve fibre bundle axially next to the first electrode arrangement (2) and comprises at least two axially spaced third electrode strips (8) extending in the circumferential direction (U) and each assuming an annular shape as well as
axially between the at least two third electrode strips (8) at least one third electrode structure (13), in each case comprising at least two third electrode surfaces (9) arranged evenly distributed in the circumferential direction.

17. The implantable device according to claim 15 and 16,
**characterised in that** the first and third electrode surfaces (4, 9) are arranged evenly distributed in the circumferential direction (U), each along a virtual circular line.

18. The implantable device according to claim 15 and 16,
**characterised in that** the first and third electrode surfaces (4, 9) each have an axial (4a, 9a) extent orientated in the circumferential direction (4U, 9U),
**in that** the extents (4a, 4U) of the first electrode surfaces (4) are identical in each case,
**in that** the extents (9a, 9U) of the third electrode surfaces (9) are identical in each case and
**in that** the extent (9U) of the third electrode surfaces (9) orientated in the circumferential direction is greater than the extent (4U) of the first electrode surfaces (4) orientated in the circumferential direction.

19. The implantable device according to claim 18,
**characterised in that** the axial extents (4a, 4U) of the first and third electrode surfaces (4, 9) are identical.

20. The implantable device according to an one of claims 16 to 19,
**characterised in that** the carrier substrate surface (1') on which the first and the third electrode arrangements (2, 7) is applied, is designed contiguously in one piece, i.e. without breaks.

21. The implantable device according to any one of claims 16 to 20,
**characterised in that** the axial distance between the first electrode strips (5) is selected to be greater than or equal to the axial distance between the third electrode strips (8) and
**in that** the axial distance between the third electrode strips measures between 0.5 cm and 3 cm, preferably between 0.75 cm and 1.25 cm.

22. The implantable device according to any one of claims 16 to 21,
**characterised in that** the shape and size of the first and the third electrode strips (5, 8) are identical,
**in that** the surface size of respectively the first and the third electrode surface (4, 9) are smaller than the surface size of respectively the first and third electrode strips (5, 8), preferably smaller than one quarter of the surface size of the first or third electrode strips (5, 8).

23. The implantable device according to any one of claims 16 to 22,
**characterised in that** the first electrode surfaces (4) are made of a metallic material, preferably iridium oxide, which has a higher charge transfer capacity than a material of which the third electrode surfaces (9) consist.

24. The implantable device according to claim 23,
**characterised in that** the metallic material of the first electrode surfaces (4) is iridium oxide and/or
**in that** the material of the third electrode surfaces (9) is a metallic material, preferably platinum, or an electrically conductive polymer.

25. The implantable device according to any one of claims 16 to 24,
**characterised in that** the first and the third electrode device (2, 7) can in each case be operated as a tripolar electrode arrangement, i.e. the first and the third electrode strips can in each case be polarised to the opposite polarity of the first and third electrode structure (3, 13).

26. The implantable device according to any one of claims 16 to 25,
**characterised in that** the first electrode arrangement (7) envisages at least one optical waveguide device (10) which comprises at least two separate optical waveguide openings (11) arranged distributed in the circumferential direction (U).

27. The implantable device according to claim 26, **characterised in that** the at least two separate optical waveguide openings (11) are arranged evenly distributed along a virtual circular line and
**in that** the optical waveguide openings (11) each have an axial (11a) and a circumferentially (11U) orientated first extent, which correspond to the those extents (9a, 9U) of the second electrode surfaces (9).

28. The implantable device according to any one of claims 16 to 27,
**characterised in that** the first electrode surfaces (4) as well as the first electrode strips (5) of the first electrode arrangement (2) as well as the third electrode surfaces (9) as well as the third electrode strips (8) of the third electrode arrangement (7) are each applied to the carrier substrate surface (1') so that they do not project beyond the carrier substrate surface (1').

29. The implantable device according to any one of claims 1 to 28,
**characterised in that** the carrier substrate (1) is made of at least one biocompatible polymer and at least in parts on the straight cylindrical carrier substrate surface (1') facing the nerve fibre bundle has an active substance that inhibits inflammatory reactions.

30. The implantable device according to any one of claims 1 to 29,
**characterised in that** the carrier substrate (1) contains biocompatible polymer.

31. The implantable device according to claim 30 as well as claim 16,
**characterised in that** the first and/or the third electrode strip (5, 8) each have at least one local opening and **in that** the first and/or the third electrode strips (5, 8) are connected flat with the carrier substrate surface (1') in such a way that the polymer at least partially penetrates the at least one opening.

32. The implantable device according to at least one of claims 1 to 31,
**characterised in that** on the rear side of the carrier substrate surface (1') on the carrier substrate (1) at least two reference electrode surfaces (12) are applied.

33. The implantable device according to any one of claims 1 to 32,
**characterised in that** in the area of the straight cylindrical carrier substrate surface (1') facing the nerve fibre bundle the biocompatible carrier substrate (1) has axially opposite marginal areas (14) at which the carrier substrate (1) has a greater substrate thickness than in the remaining carrier substrate area and
**in that** the marginal areas (14) have rounded marginal edges.

34. The implantable device according to any one of claims 1 to 33,
**characterised in that** in a carrier substrate area which cannot be applied in a cuff-like manner about the nerve fibre bundle, the biocompatible carrier substrate (1) has at least one opening (15) fully penetrating the carrier substrate.

35. The implantable device according to claim 34,
**characterised in that** the at least one opening (15) is at least in parts enveloped with a metallic material.

36. The implantable device according to claim 16 and 30,
**characterised in that** the first and/or the third electrode strips (5, 8) each have a metallic electrode strip substrate with a flat upper and lower side with at least one structural element locally projecting beyond the upper side in an orthogonal manner,
**in that** the flat upper surface of the metallic electrode strip substrate is orientated in parallel to the carrier substrate surface and
**in that** the electrode strip substrate is fully surrounded by the biocompatible polymer with the exception of a surface area of the at least one structural element which is orientated toward the carrier substrate surface and does not project beyond it.

37. The implantable device according to claim 36,
**characterised in that** at least between the lower side of the electrode strip substrate and the biocompatible polymer of the carrier substrate an adhesion promoter layer or adhesion promotor layer device is introduced.

38. The implantable device according to claim 36 or 37, **characterised in that** the one surface area of the at least one structural element or a plane assigned to the surface area is orientated in parallel to the carrier substrate surface, **in that** the surface area is arranged in a freely accessible manner from the sides of the carrier substrate surface and **in that** the at least one structural element is connected in one piece to the electrode strip substrate.

39. The implantable device according to any one of claims 36 to 38,
**characterised in that** a plurality of geometrically evenly distributed and identically designed structural elements is provided on the upper side of the metallic electrode strip substrate.

40. The implantable device according to any one of claims 36 to 39,
**characterised in that** the at least one structural element is designed in a column, rib, sleeve or web-like manner.

## Revendications

1. Système implantable pour la saisie localement sélective de signaux électriques neuronaux, qui se propagent longitudinalement au moins d'une fibre nerveuse contenue dans un faisceau de fibres nerveuses, ainsi que pour la simulation électrique sélective d'au moins une fibre nerveuse, comprenant les composants suivants :
- système d'électrodes implantable (E) qui est disposé sur un substrat de support (1) biocompatible applicable comme un manchon autour du faisceau de fibres nerveuses, qui comporte une surface de substrat de support (1') en forme de cylindre droit, orientée tournée à l'état implanté vers le faisceau de fibres nerveuses, sur laquelle est disposé un premier système d'électrodes (2) pour la saisie localement sélective des signaux électriques neuronaux et la simulation électrique sélective d'au moins une fibre nerveuse, et sur laquelle est disposé un deuxième système d'électrodes (12) pour la saisie d'un signal d'électrocardiogramme (ECG),
- une unité d'exploitation/de commande (A/S) reliée ou pouvant être reliée de façon électro-conductrice au système d'électrodes implantable (E), dans laquelle les signaux électriques neuronaux saisis de façon localement sélective ainsi que le signal d'ECG peuvent être exploités résolus dans le temps de telle sorte qu'un signal de temps neuronal peut être dérivé en corrélation avec un paramètre physiologique, de préférence la pression sanguine,
- une première unité de comparateur (K1) reliée à l'unité d'exploitation/de commande (A/S) dans laquelle un décalage de temps relatif caractéristique entre le signal d'ECG et le signal de temps neuronal en corrélation avec le paramètre physiologique peut être déterminé,
- un premier générateur de fonctions (F1) relié à l'unité d'exploitation/de commande (A/S), qui génère, à l'intérieur d'une fenêtre de temps déterminée par l'unité d'exploitation/de commande (A/S) en prenant pour base le décalage de temps relatif, un signal de simulation composé d'une pluralité n d'impulsions individuelles (EP) et adapté en phase et en courbe d'amplitude temporelle au signal de temps neuronal dérivé, en corrélation avec le paramètre physiologique, ainsi
- qu'un premier convertisseur signal-courant (SSW1) relié directement ou indirectement au premier générateur de fonctions (F1) et au premier système d'électrodes (2), qui dirige le signal de simulation pour la simulation sélective électrique d'au moins une fibre nerveuse vers le premier système d'électrodes (2),
sachant que l'unité d'exploitation/de commande (A/S), la première unité de comparateur (K1), le premier générateur de fonctions (F1) ainsi que le premier convertisseur signal-courant (SSW1) sont réunis sous la forme d'un module implantable.

2. Système implantable selon la revendication 1, **caractérisé en ce qu'**au moins une deuxième unité de comparateur (K2) est reliée à l'unité d'exploitation ((A/S),
**en ce que** la deuxième unité de comparateur (K2) compare à un signal de référence au moins un niveau de signal, qui est attribué au signal de temps neuronal en corrélation avec le paramètre physiologique et génère une valeur de niveau différentielle, et
**en ce que** l'unité d'exploitation/de commande (A/S) détermine au moins la courbe d'amplitudes temporelle du signal de simulation sur la base de la valeur de niveau différentielle.

3. Système implantable selon la revendication 1 ou 2,
**caractérisé en ce que** le système d'électrodes implantable (E) comprend sur la surface de substrat de support (1') de forme cylindrique droite orientée tournée à l'état implanté vers le faisceau de fibres nerveuses, un troisième système d'électrodes (7) pour inhiber les signaux neuronaux électriques se propageant de façon unidirectionnelle le long du faisceau de fibres nerveuses.

4. Système implantable selon l'une des revendications 1 à 3,
**caractérisé en ce que** le module implantable dispose d'un accumulateur d'énergie électrique (ES) et/ou d'une unité d'alimentation de signaux et d'énergie à base d'induction (SES), qui est reliée électriquement au moins à l'unité d'exploitation/de commande (A/S).

5. Système implantable selon l'une quelconque des revendications 1 à 4,
**caractérisé en ce qu'**un capteur d'accélération (BS) est intégré dans le module implantable et/ou un capteur d'accélération (BS') séparé du module, constitué implantable est prévu et **en ce que** le capteur d'accélération (BS, BS') génère des informations d'accélération et est relié électriquement à l'unité d'exploitation/de commande (A/S).

6. Système implantable selon l'une quelconque des revendications 1 à 5,
**caractérisé en ce que** le premier générateur de fonctions (F1) est en mesure de produire des impulsions individuelles qui sont respectivement composées d'une partie de signal polarisante et repolarisante en forme d'impulsion rectangulaire et **en ce qu'**un premier modulateur (M1) est connecté entre le premier générateur de fonctions (F1) et le premier convertisseur signal-courant (SSW1), qui amplifie et lisse dans le temps par rapport à la partie de signal polarisante un tracé de flancs de signal attribué à la partie de signal en forme d'impulsion rectangulaire, repolarisante et unifie une intensité de signal respectivement attribuée aux deux parties de signal.

7. Système implantable selon l'une quelconque des revendications 3 à 6,
**caractérisé en ce qu'**un deuxième générateur de fonctions (F2) relié à l'unité d'exploitation/de commande (A/S) est prévu, qui génère dans le temps un signal de blocage électrique avant et/ou pendant la fenêtre de temps déterminée, et
**en ce que** le deuxième générateur de fonctions (F2) est directement ou indirectement relié à un deuxième convertisseur signal-courant (SSW2), qui achemine le signal de blocage électrique au troisième système d'électrodes (7).

8. Système implantable selon la revendication 7,
**caractérisé en ce que** le deuxième générateur de fonctions (F2) est en mesure de produire des impulsions individuelles qui sont respectivement composées d'une partie de signal polarisante et repolarisante en forme d'impulsion rectangulaire et **en ce qu'**un deuxième modulateur (M2) est connecté entre le deuxième générateur de fonctions (F2) et le deuxième convertisseur signal-courant (SSW2), qui amplifie et lisse dans le temps par rapport à la partie de signal polarisante un tracé de flancs de signal attribué à la partie de signal en forme d'impulsion rectangulaire, repolarisante et unifie une intensité de signal respectivement attribuée aux deux parties de signal.

9. Système implantable selon la revendication 8,
**caractérisé en ce que** le deuxième générateur de fonction (F2) ainsi que le deuxième module (M2) sont intégrés dans le module implantable.

10. Système implantable selon la revendication 6 ou 8,
**caractérisé en ce que** le premier ou deuxième modulateur (M1, M2) est constitué de telle sorte que pur chaque impulsion individuelle, la partie de signal polarisante peut être séparée de la partie de signal repolarisante par un niveau de signal nul pouvant être généré par le premier modulateur (M1).

11. Système implantable selon l'une quelconque des revendications 1 à 10,
**caractérisé en ce qu'**une unité de mesure d'impédance d'électrodes (EM) fait partie du module implantable ou est placée sur le substrat de support (1) et est reliée au système d'électrodes (E) ainsi qu'à l'unité d'exploitation/de commande (A/S) et **en ce que** l'unité de mesure d'impédance d'électrodes (EM) procède à une mesure d'impédance au moins aux électrodes du premier système d'électrodes (2) entre chacune des n impulsions individuelles.

12. Système implantable selon la revendication 11,
**caractérisé en ce qu'**un dispositif de dépolarisation électrique (EE) fait partie du module implantable ou est placé sur le substrat de support (1) et est relié au système d'électrodes (E) ainsi qu'à l'unité d'exploitation/de commande (A/S), et
**en ce que** la dispositif de dépolarisation (EE) en cas d'une polarisation résiduelle sur les électrodes individuelles constatée par l'unité de mesure d'impédance d'électrodes (EM) dépolarise celle-ci de façon sélective au moyen d'une sollicitation de signal électrique.

13. Système implantable selon l'une quelconque des revendications 4 à 12,
**caractérisé en ce qu'**un système de mesure de la pression sanguine séparé (SB) est prévu, qui saisit les valeurs absolues de pression sanguine, qui peuvent être acheminées par l'unité d'alimentation en signaux et en énergie (SES) à base d'induction à l'unité d'exploitation/de commande (A/S) et **en ce que** l'unité d'exploitation/de commande (A/S) active le premier générateur de fonctions (F1) pour générer le signal de simulation sur la base des valeurs absolues de pression sanguine, du signal d'ECG, du signal de temps neuronal en corrélation avec le paramètre physiologique ainsi qu'au moins d'un signal de référence.

14. Système implantable selon la revendication 2, 5 et 13,
**caractérisé en ce qu'**une unité de mémoire est prévue, de préférence du type d'une table de correspondance, est prévue qui fait partie de l'unité d'exploitation/de commande (A/S) ou est reliée à celle-ci en tant qu'unité séparée,
**en ce que** dans l'unité de mémoire au moins une des informations suivantes peut être mémorisée et appelée : valeur de niveau différentielle, informations d'accélération, pression sanguine absolue saisie.

15. Système implantable selon l'une quelconque des revendications 1 à 14,
**caractérisé en ce que** la surface de substrat de support (1') orientée tournée vers le faisceau de fibres nerveuses, de forme cylindrique droite possède une extension axiale (a) et orientée en direction périphérique (U) et est mise en place sur le premier système d'électrodes (2), qui comprend
- en succession axiale au moins trois premières structures d'électrodes (3) avec respectivement au moins deux premières surfaces d'électrodes (4) disposées dans le sens périphérique, ainsi qu'
- au moins deux premières bandes d'électrodes (5) à distance axialement l'une de l'autre s'étendant dans le sens périphérique et adoptant respectivement une forme annulaire, qui comprennent axialement des deux côtés au moins trois structures d'électrodes et peuvent être reliées ou sont reliées à l'unité d'exploitation/de commande (A/S).

16. Système implantable selon la revendication 15 ainsi que selon l'une quelconque des revendications 3 à 14,
**caractérisé en ce que** le troisième système d'électrodes (7) est disposé axialement près du premier système d'électrodes (2) en succession axiale sur la surface de substrat de support (1') de forme cylindrique droite tournée vers le faisceau de fibres nerveuses et comprend au moins deux troisièmes bandes d'électrodes (8) axialement à distance l'une de l'autre, s'étendant dans le sens périphérique (U) adoptant respectivement une forme annulaire, ainsi qu'
une troisième structure d'électrodes (13) comprenant axialement entre au moins deux troisièmes bandes d'électrodes (8) au moins une, respectivement au moins deux troisièmes surfaces d'électrodes (9) disposées uniformément réparties dans le sens périphérique.

17. Système implantable selon la revendication 15 et 16,
**caractérisé en ce que** les premières et troisièmes surfaces d'électrodes (4, 9) sont disposées uniformément réparties dans la direction périphérique (U) respectivement le long d'une ligne circulaire virtuelle.

18. Système implantable selon la revendication 15 et 16,
**caractérisé en ce que** les premières et troisièmes surfaces d'électrodes (4, 9) comportent respectivement une extension axiale (4a, 9a) et orientée dans le sens périphérique (4U, 9U),
**en ce que** les extensions (4a, 4U) des premières surfaces d'électrodes (4) sont respectivement identiques, et
**en ce que** les extensions (9a, 9U) des troisièmes surfaces d'électrodes (9) sont respectivement identiques, et
**en ce que** l'extension (9U) orientée dans le sens périphérique des troisièmes surfaces d'électrodes (9) est plus grande que l'extension (4U) orientée dans le sens périphérique des premières surfaces d'électrodes (4) .

19. Système implantable selon la revendication 18,
**caractérisé en ce que** les extensions axiales (4a, 4U) des premières et troisièmes surfaces d'électrodes (4, 9) sont identiques.

20. Système implantable selon l'une quelconque des revendications 16 à 19,
**caractérisé en ce que** la surface de substrat de support (1') est appliquée au premier et au troisième système d'électrodes (2, 7), est constitué suspendue en une seule pièce, c'est-à-dire sans interruption.

21. Système implantable selon l'une quelconque des revendications 16 à 20,
**caractérisé en ce que** la distance axiale entre les premières bandes d'électrodes (5) est choisie plus grande ou identique à la distance axiale des troisièmes bandes d'électrodes (8), et
**en ce que** la distance axiale entre les troisièmes bandes d'électrodes mesure entre 0,5 cm et 3 cm, de préférence entre 0,75 cm et 1,25 cm.

22. Système implantable selon l'une quelconque des revendications 16 à 21,
**caractérisé en ce que** la forme et la taille des premières et troisièmes bandes d'électrodes (5, 8) sont identiques,
**en ce que** les tailles de surface respectivement de la première et troisième surface d'électrodes (4, 9) sont plus petites que la taille de surface respectivement des premières ou troisièmes bandes d'électrodes (5, 8), de préférence plus petites qu'un quart de la taille de surface des premières ou troisièmes bandes d'électrodes (5, 8).

23. Système implantable selon l'une quelconque des revendication 16 à 22,
**caractérisé en ce que** les premières surfaces d'électrodes (4) sont composées d'un matériau métallique, de préférence d'oxyde d'iridium, qui dispose d'une capacité de transmission de charge plus élevée qu'un matériau composant les troisièmes surfaces d'électrodes (9).

24. Système implantable selon la revendication 23, **caractérisé en ce que** le matériau métallique des premières surfaces d'électrodes (4) est de l'oxyde d'iridium et/ou
**en ce que** le matériau des troisièmes surfaces d'électrodes (9) est un matériau métallique, de préférence du platine ou un polymère électro-conducteur.

25. Système implantable selon l'une quelconque des revendications 6 à 24,
**caractérisé en ce que** le premier et troisième système d'électrodes (2, 7) peuvent être utilisés respectivement en tant que système d'électrodes tripolaire, c'est-à-dire que les premières et troisièmes bandes d'électrodes sont respectivement polarisables en polarité opposée à la première et troisième structure d'électrodes (3, 13).

26. Système implantable selon l'une quelconque des revendication 16 à 25,
**caractérisé en ce que** le troisième système d'électrodes (7) prévoit au moins un système de fibres optiques (10), qui comprend au moins deux ouvertures de fibres optiques (11) séparées disposées réparties dans le sens périphérique (U).

27. Système implantable selon la revendication 26
**caractérisé en ce qu'**au moins deux ouvertures de fibres optiques (11) séparées sont disposées uniformément réparties le long d'une ligne circulaire virtuelle, et
**en ce que** les ouvertures de fibres optiques (11) possèdent respectivement une extension axiale (11a) et une extension orientée dans le sens périphérique (11U), qui correspondent aux extensions (9a, 9U) des deuxièmes surfaces d'électrodes (9).

28. Système implantable selon l'une quelconque des revendications 16 à 27,
**caractérisé en ce que** les premières surfaces d'électrodes (4) ainsi que les premières bandes d'électrodes (5) du premier système d'électrodes (2) ainsi que les troisièmes surfaces d'électrodes (9) et les troisièmes bandes d'électrodes (8) du troisième système d'électrodes (7) sont respectivement disposées sur la surface de substrat de support (1') de telle sorte qu'elles ne dépassent pas la surface de substrat de support (1').

29. Système implantable selon l'une quelconque des revendications 1 à 28,
**caractérisé en ce que** le substrat de support (1) est fabriqué dans au moins un polymère biocompatible et comporte au moins par zone une substance active inhibant les réactions inflammatoires sur la surface de substrat de support (1') de forme cylindrique droite tournée vers le faisceau de fibres nerveuses.

30. Système implantable selon l'une quelconque des revendications 1 à 29,
**caractérisé en ce que** le substrat de support (1) contient un polymère biocompatible.

31. Système implantable selon la revendication 30 et la revendication 16,
**caractérisé en ce que** les premières et/ou troisièmes bandes d'électrodes (5, 8) disposent respectivement d'au moins une ouverture locale et **en ce que** les premières et/ou troisièmes bandes d'électrodes (5, 8) sont reliées alignées avec la surface de substrat de support (1') de telle sorte que le polymère traverse au moins une ouverture au moins en partie.

32. Système implantable selon l'une quelconque des revendications 1 à 31,
**caractérisé en ce qu'**au moins deux surfaces d'électrodes de référence (12) sont mises en place à l'arrière de la surface de substrat de support (1') sur le substrat de support (1).

33. Système implantable selon l'une quelconque des revendications 1 à 32,
**caractérisé en ce que** le substrat de support (1) biocompatible comporte dans la zone de la surface de substrat de support (1') de forme cylindrique droite, orientée tournée vers le faisceau de fibres nerveuses des zones de bordure (14) s'opposant respectivement axialement sur lesquelles le substrat de support (1) dispose d'une épaisseur de substrat plus importante que dans la zone restante de substrat de support, et
**en ce que** les zones de bordure (14) disposent de bords de bordure arrondis.

34. Système implantable selon l'une quelconque des revendications 1 à 33,
**caractérisé en ce que** le substrat de support biocompatible (1) comporte, dans une zone de substrat de support qui ne peut pas être appliquée autour du faisceau de fibres nerveuses à la manière d'un manchon, au moins une ouverture (15) traversant complètement le substrat de support.

35. Système implantable selon la revendication 34,
**caractérisé en ce qu'**au moins une ouverture (15) est enveloppée au moins par zone d'un matériau métallique.

36. Système implantable selon la revendication 16 et 30,
**caractérisé en ce que** les premières et/ou troisièmes bandes d'électrodes (5, 8) comportent respectivement un substrat de bandes d'électrodes métallique avec une face supérieure et inférieure plane, avec au moins un élément de structure dépassant orthogonalement, localement la face supérieure,
**en ce que** la surface plane du substrat de d'électrodes métallique est orientée parallèlement à la surface de substrat de support, et
**en ce que** le substrat de bandes d'électrodes est complètement entouré du polymère biocompatible à l'exception d'une zone de surface d'au moins un élément de structure, qui est orientée tournée vers la surface de substrat de support et ne dépasse pas celle-ci.

37. Système implantable selon la revendication 36,
**caractérisé en ce qu'**une couche d'agent adhésif ou un système de couche d'agent adhésif est appliqué au moins entre la face inférieure du substrat de bande d'électrodes et le polymère biocompatible du substrat de support.

38. Système implantable selon la revendication 36 ou 37,
**caractérisé en ce qu'**une zone de surface d'au moins un élément de structure ou un plan attribué à la zone de surface est orienté parallèlement à la surface de substrat de support, **en ce que** la zone de surface des côtés de la surface de substrat de support est disposée librement accessible et **en ce qu'**au moins un élément de structure est relié en une seule pièce aux substrats de bandes d'électrodes.

39. Système implantable selon l'une quelconque des revendications 36 à 38,
**caractérisé en ce qu'**une pluralité d'éléments de structure disposés uniformément répartis de façon géométrique sur la face supérieure du substrat de bandes d'électrodes métallique et constitués de façon identique est prévue.

40. Système implantable selon l'une quelconque des revendications 36 à 39,
**caractérisé en ce qu'**au moins un élément de structure est constitué en forme de colonne, de membrure, de manchons ou de nervure.
